# EUROPEAN PATENT SPECIFICATION

(11) **EP 1 021 186 B1**
(45) Date of publication and mention of the grant of the patent: **29.06.2005**
(21) Application number: 97936297.7
(22) Date of filing: 01.08.1997
(51) Int. Cl.: A61K 31/52, C07D 473/40, C07D 473/38, C07D 473/36, C07D 473/34, C07D 473/32, C07D 473/30, C07D 473/24, C07D 473/22, C07D 473/20, C07D 473/18

(54) **PURINE INHIBITORS OF CYCLIN DEPENDENT KINASE 2 AND IkappaB-alpha**
PURINHEMMER VON CYCLIN-ABHÄNGIGER KINASE 2 UND IkappaB-alpha
INHIBITEURS PURIQUES DE LA KINASE 2 ET IkappaB-alpha DEPENDANT DE LA CYCLINE

(30) Priority: 02.08.1996 US 692012
(43) Date of publication of application: 26.07.2000
(73) Proprietor: CV THERAPEUTICS, INC., Palo Alto, CA 94304 (US)
(72) Inventor: LUM, Robert, T., Palo Alto, CA 94306 (US); BLUM, Cheri, Lynn, Alameda, CA 94501 (US); MACKMAN, Richard, San Carlos, CA 94070 (US); WICK, Michael, M., Chestnut Hill, MA 02146 (US); SCHOW, Steven, R., Redwood City, CA 94065 (US)
(74) Representative: Vossius, Volker, Dr.
(86) International application number: PCT/US1997/013386
(87) International publication number: WO 1998/005335

(56) References cited:
- WO-A-93/17020
- WO-A-97/16452
- WO-A-97/18212
- WO-A-97/20842
- US-A- 4 028 358
- US-A- 4 405 781
- US-A- 5 498 819
- US-A- 5 508 277
- EUR. J. BIOCHEM., 1994, Volume 224, VESELY et al., "Inhibition of Cyclin-Dependent Kinases by Purine Analogues", pages 771-786.
- JOURNAL OF THE AMERICAN CHEMICAL SOCIETY, 20 July 1959, Volume 81, BRESHEARS et al., "The Aminolysis of Certain Chlorosubstituted Purines", pages 3789-3792.

## Description

This invention concerns 2,6,9-trisubstituted purines that are selective inhibitors of cell cycle kinases and inhibitors of cell proliferation. The purines are useful for example in - treating autoimmune diseases, e.g. rheumatoid arthritis, lupus, type I diabetes, multiple sclerosis, etc., in treating cancer, cardiovascular disease, such as restenosis, host vs graft disease, gout, polycystic kidney disease and other proliferative diseases whose pathogenesis involves abnormal cell proliferation.

This invention also concerns 2,6,9-trisubstituted purines that have been discovered to be potent and specific inhibitors of IκB-α kinase which prevents signal induced NF-κB activation and cytokine synthesis in vitro and in vivo. Such inhibitors are expected to inhibit the synthesis of cytokines and adhesion proteins whose synthesis is transcriptionally regulated by NF-κB. Proinflammatory cytokines such as IL-1, IL-6, TNF and adhesion proteins (e.g. ICAM, VCAM and selections) belong to this class of molecules and have been implicated in the pathogenesis of inflammatory diseases. Thus a potent inhibitor of IκB-α kinase is useful in the clinical management of diseases where NF-κB activation is required for disease induction.

In the past few years, advances in molecular and cellular biology have contributed to our understanding of the mechanisms of cell proliferation end of specific events that occur during progression of cells through mitosis. *E*.*g*., "Progress in Cell Cycle Research" Vol. 1, Eds. L. Meijer, S. Guidet and H.Y.L. Tung; Plenum Press, New York, 1995. These studies have shown that progression through the cell cycle is controlled by a family of serine/threonine kinases called cyclin dependent kinases. These enzymes contain (a) a catalytic protein called cyclin dependent kinase (CDK) that uses ATP as a substrate and (b) a regulatory protein called cyclin. Different cyclin-CDK combinations control events such as growth, DNA replication and cell division. One key member of the CDK family of enzymes is CDK2. CDK2 activity has been shown to be essential for mammalian cell cycle progression at the G1/S boundary. Microinjection of antibodies directed against CDK2 blocks the progression of human diploid fibroblasts into the S phase of the cell cycle. Expression of a CDK2 dominant negative mutant in human osteosarcoma cells has a similar effect. Together, these studies indicate that inhibition of cellular CDK2 activity will prevent progression of cells through the mitotic cycle and induce growth arrest prior to the S phase. Consistent with this view, in vitro studies with olomoucine (2-(hydroxyethylamino)-6-benzylamino-9-methylpurine), have shown that it is specific inhibitor of CDK2 with an IC₅₀ of approximately 2.1 µg/ml J. Vesely, et al.; Eur. J. Biochem 224, 771-786 (1994), L. Meijer "Chemical Inhibitors of Cyclin-Dependent Kinases" pp. 351-356 in "Progress in Cell Cycle Research Vol.1, Eds. L. Meijer, S. Guidet and H.Y.L. Tung; Plenum Press, New York, 1995. In vivo studies using mammalian cells in culture have shown that olomoucine inhibits cell proliferation at an approximate concentration of 50 µg/ml.

In this invention, we have developed several compounds whose biological activity is considerably more potent than olomoucine. In vivo studies using mammalian cells indicate that some of the disclosed compounds inhibit cell proliferation at concentrations that are significantly lower than olomoucine.

Recently an IκB-α kinase activity has been described in the cytoplasm of stimulated human umbilical vein endothelial cells (Bennett et al. (1996) J. Biol. Chem 271, 19680-19688). Some of the compounds of this invention have been identified as potent and specific inhibitors of IκB-α kinase which prevents signal induced NF-κB activation and cytokine synthesis in vitro and in vivo. The activation of the heterodimeric transcription factor NF-κB is a complex process. In unstimulated cells, the NF-κB (p50/p65) heterodimer is located in the cytosol where it is complexed with an inhibitory subunit IκB-α, IκB-α binds to NF-κB thus masking its nuclear localization signal and preventing translocation to the nucleus. Upon stimulation of cells with a variety of signals (e.g. lipopolysaccharide) IκB-α is rapidly phosphorylated, uniquitinated and degraded by the proteasome. Degradation of IκB-α allows the translocation of NF-κB to the nucleus where it activates transcription of a number of inflammatory response genes.

These observations suggest that IκB-α kinase is an attractive target for the identification of inhibitors that may be useful in the treatment of inflammatory diseases where NF-κB activation is required for disease induction.

Veselý et al., European Journal of Biochemistry, vol. 224, 771-786, disclose a study relating to the inhibition of cyclin-dependent kinases by purine analogues. This reference discloses inter alia 2-(2-hydroxyethylamino)-6-benzylamino-9-methylpurine.

US-A-5,508,277 relates to pyrimidine compounds for use as drugs, which compounds and physiologically tolerable salts thereof may be used in therapeutics, particularly for suppressing tumour cell resistance to antineoplastic agents.

US-A-5,498,819 relates to griseolic acid derivatives having various groups attached to the sugar part in place of the adenine group of griseolic acid itself. These groups are all purine derivatives or ring-opened analogs. The compounds are useful as inhibitors of phosphodiesterases.

WO-A-93/17020 relates to certain purine nucleoside analogues containing a carbocyclic ring in place of the sugar residue, salts, esters and pharmaceutically acceptable derivatives thereof, processes for their preparation, pharmaceutical formulations containing them and to the use of such compounds in therapy, particularly the treatment or prophylaxis of certain infections.

### SUMMARY OF THE INVENTION

It is an object of this invention to provide 2,6,9-trisubstituted purine compounds, which inhibit the cyclin dependent kinase 2.

It is another object of this invention to provide 2,6,9-trisubstituted purine compounds, which are useful for inhibiting cell proliferation.

This invention also relates to a pharmaceutical composition, which comprises a 2,6,9-trisubstituted purine compound and a pharmaceutically acceptable carrier suitable for inhibiting cell proliferation in mammals.

In one aspect, this invention provides a compound according to claim 1 having the general formula: 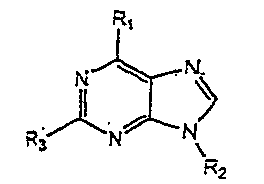 or a pharmaceutically acceptable salt thereof wherein
R₁ is halogen;
R₂ is C₂ to C₁₀ alkyl optionally substituted with one, two or three groups selected from hydroxy, halogen, and -C(O)R;
R and R' independently represent hydrogen, or
   lower alkyl optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
   aryl or heteroaryl optionally substituted with one, two or three groups selected from lower alkyl, alkoxy, halogen, mercapto, alkylthio, acetylene, amino, mono- or dialkylamino, -C(O)NH-alkyl, -C(O)N-dialkyl, -C(O)O-alkyl, -C(O)alkyl, hydroxy, aryl, aryloxy, heteroaryl, nitro or cyano; and
R₃ is halogen, -NR₄R₅ or a moiety having the general formula: 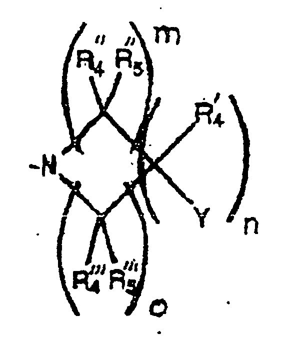
where
m, n, and o independently represent 1, 2 or 3;
Y is -NR₄R₅, hydroxy, mercapto, -OR, alkylthiol; and
R₄, R₄', R₄", R₄''', R₅, R₅" and R₅''' are each independently hydrogen, hydroxy,
   lower alkyl, optionally substituted with one, two or three groups selected from hydroxy, NRR', or
   aryl, heteroaryl, or arylalkyl; or
Y and R₄' together may be a single oxygen atom; or
R₄" and R₅" together may be a single oxgen atom; or
R₄''' and R₅''' together may be a single oxygen atom.

In another aspect, this invention provides a compound according to claim 7 having the general formula: 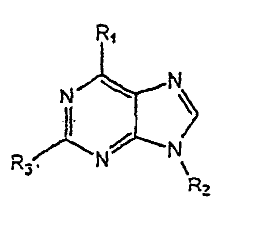 or a pharmaceutically acceptable salt thereof wherein
R₁ is -NHR₁';
R₁' is quinolin-3-yl or quinolin-6-yl; or
R₁' is benzyl where the ring portion is substituted with one, two or three groups selected from halogen, -OR, thienyl, nitro, pyridyl, piperonyl, or
   phenyl, where the phenyl is unsubstituted or substituted with trifluoromethyl, halogen,
   lower alkyl, lower alkoxy, hydroxy, amino, mercapto, alkylthio, nitro or cyano; or
R₁' is phenyl substituted with one, two or three groups selected from bromo, iodo, -OR", thienyl, pyridyl, piperonyl, or
   phenyl, where the phenyl is unsubstituted or substituted with trifluoromethyl, halogen,
   lower alkyl, lower alkoxy, hydroxy, amino, mercapto, alkylthio, nitro or cyano;
R₂ is C₂ to C₁₀ alkyl optionally substituted with one, two or three groups selected from hydroxy, halogen, and -C(O)R;
R and R' independently represent hydrogen, or
   lower alkyl, optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
   aryl or heteroaryl optionally substituted with one, two or three groups selected from lower alkyl, alkoxy, halogen, mercapto, alkylthiol, acetylene, amino, mono- or dialkylamino, -C(O)NH-alkyl, -C(O)N-dialkyl, -C(O)O-alkyl, - C(O)alkyl, hydroxy, aryl, aryloxy, heteroaryl nitro or cyano;
R" represents hydrogen, or
   C₂ to C₁₀ alkyl, optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
   aryl or heteroaryl optionally substituted with one, two or three groups selected from lower alkyl, alkoxy, halogen, mercapto, alkylthiol, acetylene, amino, mono- or dialkylamino, -C(O)NH-alkyl, -C(O)N-dialkyl, -C(O)O-alkyl, - C(O)alkyl, hydroxy, aryl, aryloxy, heteroaryl, nitro or cyano;
R₃ is halogen, -NR₄R₅ or a moiety having the general formula: 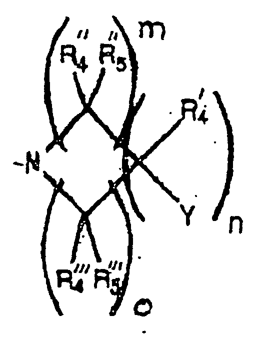
where
m, n, and o independently represent 1, 2 or 3;
Y is -NR₄R₅, hydroxy, mercapto, -OR, alkylthio ; and
R₄, R₄', R₄", R₄''', R₅, R₅" and R₅''' independently are hydrogen, hydroxy, lower alkyl optionally substituted with one, two or three groups selected from hydroxy, -NRR', or aryl, heteroaryl, or arylalkyl; or
Y and R₄' together may be a single oxygen atom; or
R₄" and R₅" together may be a single oxgen atom; or
R₄''' and R₅''' together may be a single oxygen atom.

Preferred embodiments of the compound of claim 1 are set forth in claims 2 to 6. Furthermore, preferred embodiments of the compound of claim 7 are set forth in claims 8 to 23.

In another aspect, this invention provides the use of a therapeutically effective amount of a compound as claimed in any one of claims 7 to 25 for the preparation of a pharmaceutical composition suitable for inhibiting cell proliferation in mammals. Cell proliferation disorders are e.g. rheumatoid arthritis, lupus, type I diabetes, multiple sclerosis, cancer, restenosis, host graft disease, and gout.

In yet another aspect, this invention provides a pharmaceutical composition comprising a compound of any one of claims 7 to 25 in an admixture with one or more pharmaceutical excipients.

In still another aspect, this invention provides an antifungal composition useful for treating fungal infections in humans, animals and plants comprising a compound of any one of claims 7 to 25.

### DESCRIPTION OF THE FIGURE

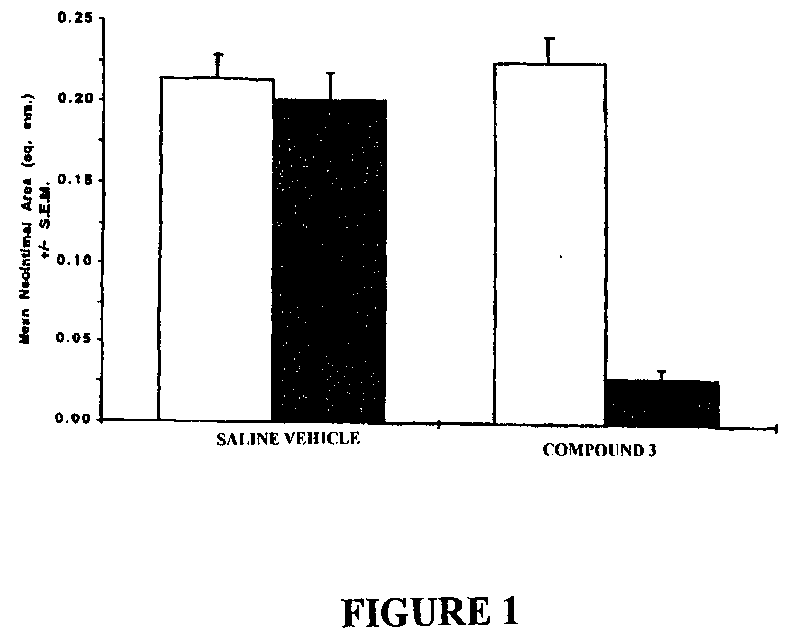

Figure 1 is a plot of the mean neointimal area of a rat carotid artery treated with a saline vehicle and treated with compound 3 prepared according to Example 2 wherein the unshaded bar represents the untreated section of the carotid artery and the shaded bar represents the treated section of the carotid artery.

The following are definitions for certain terms used herein.

"Halogen" refers to fluorine, bromine, chlorine, and iodine atoms.

"Hydroxyl" refers to the group -OH.

"Thiol" or "mercapto" refers to the group -SH.

"Lower alkyl" refers to a cyclic, branched or straight chain, alkyl group of one to ten carbon atoms. This term is further exemplified by such groups as methyl, ethyl, n-propyl, i-propyl, n-butyl, t-butyl, i-butyl (or 2-methylpropyl), cyclopropylmethyl, i-amyl, n-amyl, hexyl and the like.

"Substituted lower alkyl" refers to lower alkyl as just described including one or more groups such as hydroxyl, thiol, alkylthiol, halogen, alkoxy, amino, amido, carboxyl, cycloalkyl, substituted cycloalkyl, heterocycle, cycloheteroalkyl, substituted cycloheteroalkyl, acyl, carboxyl, aryl, substituted aryl, aryloxy, hetaryl, substituted hetaryl, aralkyl, heteroaralkyl, alkyl alkenyl, alkyl alkynyl, alkyl cycloalkyl, alkyl cycloheteroalkyl, cyano. These groups may be attached to any carbon atom of the lower alkyl moiety.

"Alkyl alkenyl" refers to a group -R-CR'=CR''' R'''', where R is lower alkyl, or substituted lower alkyl, R', R''', R'"'may independently be hydrogen, halogen, lower alkyl, substituted lower alkyl, acyl, aryl, substituted aryl, hetaryl, or substituted hetaryl as defined below.

"Alkyl alkynyl" refers to groups -RC≡CR' where R is lower alkyl or substituted lower alkyl, R' is hydrogen, lower alkyl, substituted lower alkyl, acyl, aryl, substituted aryl, hetaryl, or substituted hetaryl as defined below.

"Alkoxy" denotes the group -OR, where R is lower alkyl, substituted lower alkyl, acyl, aryl, substituted aryl, aralkyl, substituted aralkyl, heteroalkyl, heteroarylalkyl, cycloalkyl, substituted cycloalkyl, cycloheteroalkyl, or substituted cycloheteroalkyl as defined.

"Alkylthio" denotes the group -SR, -S(O)ₙ₌₁₋₂-R, where R is lower alkyl, substituted lower alkyl, aryl, substituted aryl, aralkyl or substituted aralkyl as defined herein.

"Acyl" denotes groups -C(O)R, where R is hydrogen, lower alkyl substituted lower alkyl, aryl, substituted aryl and the like as defined herein.

"Aryloxy" denotes groups -OAr, where Ar is an aryl, substituted aryl, heteroaryl, or substituted heteroaryl group as defined herein.

"Amino" denotes the group NRR', where R and R' may independently by hydrogen, lower alkyl, substituted lower alkyl, aryl, substituted aryl, hetaryl, or substituted hetaryl as defined herein or acyl.

"Amido" denotes the group -C(O)NRR', where R and R' may independently by hydrogen, lower alkyl, substituted lower alkyl, aryl, substituted aryl, hetaryl, substituted hetaryl as defined herein.

"Carboxyl" denotes the group -C(O)OR, where R is hydrogen, lower alkyl, substituted lower alkyl, aryl, substituted aryl, hetaryl, and substituted hetaryl as defined herein.

"Aryl" or "Ar" refers to an aromatic carbocyclic group having at least one aromatic ring (*e*.*g*., phenyl or biphenyl) or multiple condensed rings in which at least one ring is aromatic, (*e*.*g*., 1,2,3,4-tetrahydronaphthyl, naphthyl, anthryl, or phenanthryl).

"Substituted aryl" refers to aryl optionally substituted with one or more functional groups, *e.g.*, halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heterocycle" refers to a saturated, unsaturated, or aromatic carbocyclic group having a single ring (*e*.*g*., morpholino, pyridyl or furyl) or multiple condensed rings (*e*.*g*., naphthpyridyl, quinoxalyl, quinolinyl, indolizinyl or benzo[b]thienyl) and having at least one hetero atom, such as N, O or S, within the ring, which can optionally be unsubstituted or substituted with, *e*.*g*., halogen, lower alkyl, lower alkoxy, alkylthio, acetylene, amino, amido, carboxyl, hydroxyl, aryl, aryloxy, heterocycle, hetaryl, substituted hetaryl, nitro, cyano, thiol, sulfamido and the like.

"Heteroaryl" or "hetar" refers to a heterocycle in which at least one heterocyclic ring is aromatic.

"Substituted heteroaryl" refers to a heterocycle optionally mono or poly substituted with one or more functional groups, *e*.*g*., halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Aralkyl" refers to the group -R-Ar where Ar is an aryl group and R is lower alkyl or substituted lower alkyl group. Aryl groups can optionally be unsubstituted or substituted with, *e*.*g*., halogen, lower alkyl, alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Heteroalkyl" refers to the group -R-Het where Het is a heterocycle group and R is a lower alkyl group. Heteroalkyl groups can optionally be unsubstituted or substituted with *e*.*g*., halogen, lower alkyl, lower alkoxy, alkylthio, acetylene, amino, amido, carboxyl, aryl, aryloxy, heterocycle, substituted heterocycle, hetaryl, substituted hetaryl, nitro, cyano, thiol, sulfamido and the like.

"Heteroarylalkyl" refers to the group -R-HetAr where HetAr is an heteroaryl group and R lower alkyl or substituted lower alkyl. Heteroarylalkyl groups can optionally be unsubstituted or substituted with, *e.g.*, halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloalkyl" refers to a divalent cyclic or polycyclic alkyl group containing 3 to 15 carbon atoms.

"Substituted cycloalkyl" refers to a cycloalkyl group comprising one or more substituents with, *e*.*g*., halogen, lower alkyl, substituted lower alkyl, alkoxy, alkylthio, acetylene, aryl, aryloxy, heterocycle, substituted heterocycle, hetaryl, substituted hetaryl, nitro, cyano, thiol, sulfamido and the like.

"Cycloheteroalkyl" refers to a cycloalkyl group wherein one or more of the ring carbon atoms is replaced with a heteroatom (*e*.*g*., N, O, S or P).

"Substituted cycloheteroalkyl" refers to a cycloheteroalkyl group as herein defined which contains one or more substituents, such as halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloalkyl" denotes the group -R-cycloalkyl where cycloalkyl is a cycloalkyl group and R is a lower alkyl or substituted lower alkyl. Cycloalkyl groups can optionally be unsubstituted or substituted with *e.g.* halogen, lower alkyl, lower alkoxy, alkylthio, acetylene, amino, amido, carboxyl, hydroxyl, aryl, aryloxy, heterocycle, substituted heterocycle, hetaryl, substituted hetaryl, nitro, cyano, thiol, sulfamido and the like.

"Alkyl cycloheteroalkyl" denotes the group -R-cycloheteroalkyl where R is a lower alkyl or substituted lower alkyl. Cycloheteroalkyl groups can optionally be unsubstituted or substituted with *e.g.* halogen, lower alkyl, lower alkoxy, alkylthio, amino, amido, carboxyl, acetylene, hydroxyl, aryl, aryloxy, heterocycle, substituted heterocycle, hetaryl, substituted hetaryl, nitro, cyano, thiol, sulfamido and the like.

If the final 2,6,9-trisubstituted purine compound of this invention contains a basic group, then an acid addition salt of the composition may be prepared. Acid addition salts of the compounds of this invention are prepared in a standard manner in a suitable solvent from the parent compound and an excess of acid, such as, but not limited to, hydrochloric, hydrobromic, sulfuric, phosphoric, acetic, maleic, succinic, or methanesulfonic. The hydrochloric salt form is especially useful.

If the final 2,6,9-trisubstituted purine compound contains an acidic group, then cationic salts of the composition may be prepared. Typically the acidic parent compound is treated with an excess of an alkaline reagent, such as, but not limited to, hydroxide, carbonate or alkoxide, containing the appropriate cation such as but not limited to, Na⁺, K⁺; Ca²⁺ and NH₄⁺ Certain of the compounds form inner salts or zwitterions which are also acceptable.

The compounds of this invention are useful in inhibiting cell proliferation in mammals including humans. The 2,6,9-trisubstituted purines are useful in for example in treating autoimmune diseases, e.g. rheumatoid arthritis, lupus, type 1 diabetes, multiple sclerosis, etc., in treating cancer, cardiovascular disease such as restenosis, host vs graft disease, gout, polycystic kidney disease and other proliferative diseases whose pathogenesis involves abnormal cell proliferation.

The use of the compounds of the present invention comprises the administration parenterally, and orally, of an effective quantity of the chosen compound of this invention, preferably dispersed in a pharmaceutical carrier. Therapeutically useful amounts of the compounds of this invention will generally range from 0.01 to 100 mg/kg, but will be readily determined by one skilled in the art depending upon the route of administration, and the age and condition of the patient. Therapeutically useful amounts of the compounds of this invention may be administered from one to ten times daily or more for acute or chronic disease. No unacceptable toxicological effects are expected when compounds of the invention are administered in accordance with the present invention.

The compounds of this invention are also useful as antiinflammatory and antifungal agents. As such, the compounds of this invention are useful for treating antiinflammatory and fungal infections in humans, animals, and fungal infections in plants.

Pharmaceutical compositions including the compounds of this invention, and/or derivatives thereof, may be formulated as solutions or lyophilized powder for parenteral administration. Powders may be reconstituted by addition of a suitable diluent or other pharmaceutically acceptable carrier prior to use. If used in liquid form the compounds of this invention are preferably incorporated into a buffered, isotonic, aqueous solution. Examples of suitable diluents are normal isotonic saline solution, standard 5% dextrose in water and buffered sodium or ammonium acetate solution. Such liquid formulations are suitable for parenteral administration, but may also be used for oral administration.

It may be desirable to add excipients such as polyvinylpyrrolidone, gelatin, hydroxycellulose, acaia, polyethylene glycol, mannitol, sodium chloride, sodium citrate or any other excipient known to one of skill in the art to pharmaceutical compositions including compounds of this invention. Alternatively, the pharmaceutical compositions may be encapsulated, tableted or prepared in an emulsion or syrup for oral administration. Pharmaceutically acceptable solid or liquid carriers may be added to enhance or stabilize the composition, or to facilitate preparation of the composition. Liquid carriers include, but are not limited to syrup, peanut oil, olive oil, glycerin, saline, alcohols and water. Solid carriers include, but are not limited to, starch, lactose, calcium sulfate, dihydrate, teffa alba, magnesium stearate or stearic acid, talc, pectin, acacia, agar or gelatin. The carrier may also include a sustained release material such as, but not limited to, glyceryl monostearate or glyceryl distearate, alone or with a wax. The amount of solid carrier varies bat, preferably, will be between about 20 mg to about L gram per dosage unit.

The pharmaceutical dosages are made using conventional techniques such as, but not limited to, milling, mixing, granulation, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly or filled into a soft gelatin capsule.

The Examples which follow serve to illustrate this invention. The Examples are intended to in no way limit the scope of this invention, but are provided to show how to make and use the compounds of this invention. In the Examples, all temperatures are in degrees Centigrade. RT indicates room temperature.

### EXAMPLE 1

The compounds of this invention are prepared by conventional methods of organic chemistry. The reaction sequence outlined in the synthesis scheme below is a general method useful for the synthesis of compounds of this invention. 2,6-dichloropurine is dissolved in butanol and the appropriate R₁ amine is added. After heating for several hours, the reaction mixture is cooled, and the compound 1 is obtained. To compound 1, is added, sodium hydride followed by R₂, and compound 2 is isolated. To compound 2, R₃ is added in solution with N-methylpyrrolidinone. The mixture is heated for an appropriate period followed by 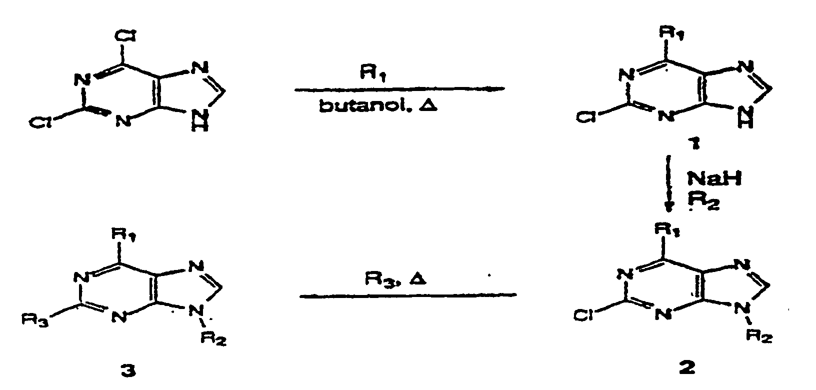 purification leading to the desired compound.

The following compound was prepared according to the method above.

### Preparation of 2-chloro-6-(4-methoxybenzylamino) purine (1).

The 2,6-dichloropurine (4.06 g, 21.5 mmol) was suspended in n-butanol (150 ml) and the 4-methoxybenzylamine was added (3.4 ml, 26 mmol). The solution turned clear and then cloudy a few minutes later. The solution was heated at 120°C for 2 hr and then cooled. The n-butanol was evaporated followed by suspension of the residue in water and diethyl ether mixture. A solution of 2N NaOH (1.3ml, 26 mmol) was added and the solution stirred for 10 min before filtration. The filtered precipitate was washed with water and a small portion of ether and then dried under vacuum. The residual liquor was left overnight and more crystals were collected the next day and washed with diethyl ether. Yield = 71 %.

### Preparation of 2-chloro-6-(4-methoxybenzylamino)-9-isopropylpurine (2)

2-chloro-6-(4-methoxybenzylamino) purine was suspended in dry DMF (5 ml) and treated with sodium hydride, 60% dispersion (82 mg, 2.06 mmol). The suspension was stirred for 30 min over which time it became a clear yellow/green solution. 2-Iodopropane (0.280 mL, 1.7 eq.) was added over 5 min and the resultant solution stirred for 2 days. Water was added and the solution extracted with ethyl acetate. The organic layer was evaporated to give the product isopropyl purine (Yield = 508 mg, 89%).

### Preparation of 2-diethanolamino-6-(4-methoxybenzylamino)-9-isopropylpurine, (3).

The purine (1.65g, 4.98 mmol) was dissolved in DMSO (12 mL) and diethanolamine (4 mL) and then heated at 140°C for 2-3 days and then at 160°C for 1 day. The solution was cooled and water saturated butanol was added (100 mL). The solution was then washed with water (3 x 50 mL), before being evaporated to give a brown oil. The residue was chromatographed over silica gel eluting with ethyl acetate, followed by 3% methanol in ethyl acetate to give the product (Yield = 730 mg, 37%) as a pale yellow oil. Yield =37%.
¹H-NMR(δ CDCl3): 7.29(br s 1H), 7.25(d, 2H), 6.94(br s. 1H), 6.83(d. 2H), 5.43(br s.<2H), 4.63(br s. 2H), 4.53(m 1H), 3.86(t. 4H), 3.76(m, 7H), 1.47(d 6H).

Table 1 identifies compounds that were prepared according to the synthesis method set forth in this Example. Those compounds, which are not encompassed by the appended claims, are given for comparative purposes only.

**TABLE 1**

| **Compounds Prepared By The Method of Example 1.** | | |
|---|---|---|
| **R1** | **R2** | **R3** |
| 4-methoxybenzylamino | 3-cyanopropyl | Cl |
| 4-methoxybenzylamino | 3-chloropropyl | Cl |
| 4-methoxybenzylamino | benzyl | Cl |
| 4-methoxybenzylamino | Methyl 4-carboxybenzyl | Cl |
| 4-methoxybenzylamino | N-phthaloylethyl | Cl |
| 4-methoxybenzylamino | isopropyl | Ethanolamine |
| 4-methoxybenzylamino | isopropyl | Diethanolamine |
| 4-methoxybenzylamino | 3-methylbutyl | Cl |
| 4-methoxybenzylamino | 2-methylpropyl | Cl |
| 4-methoxybenzylamino | cyclopentyl | Cl |
| 4-methoxybenzylamino | 3-nitrobenzyl | Cl |
| 4-methoxybenzylamino | 4-nitrobenzyl | Cl |
| 4-methoxybenzylamino | ethyl | Cl |
| 4-methoxybenzylamino | propyl | Cl |
| 4-methoxybenzylamino | 3-methylbenzyl | Cl |
| 4-methoxybenzylamino | 4-methylbenzyl | Cl |
| heptylamine | H | Cl |
| N-benzylhydroxylamine | H | Cl |
| propylamine | H | Cl |
| noradamantylamine | H | Cl |
| cyclobutylamine | H | Cl |
| 3-methoxypropylamine | H | Cl |
| 2-methoxyethylamine | H | Cl |
| cyclopentylamine | H | Cl |
| 2-amino-2-methyl-1-propanol | H | Cl |
| 4-amino-1-benzylpiperidine | H | Cl |
| heptylamine | Me | Cl |
| N-benzylhydroxylamine | Me | Cl |
| propylamine | Me | Cl |
| noradamantylamine | Me | Cl |
| cyclobutylamine | Me | Cl |
| 3-methoxypropylamine | Me | Cl |
| 2-methoxyethylamine | Me | Cl |
| cyclopentylamine | Me | Cl |
| 2-amino-2-methyl-1-propanol | Me | Cl |
| 4-amino-1-benzylpiperidine | Me | Cl |
| 2,4-dimethoxybenzylamine | Me | Cl |
| 2-methoxybenzylamine | H | Cl |
| 2-(aminomethyl)pyridine | H | Cl |
| 3,4-dimethoxyphenethylamine | H | Cl |
| 3-(aminomethyl)pyridine | H | Cl |
| 4-(aminomethyl)pyridine | H | Cl |
| 6-amino-1-hexanol | H | Cl |
| phenethylamine | H | Cl |
| 2-aminobenzothiazole | H | Cl |
| 2,4-dimethoxybenzylamine | H | Cl |
| 2-methoxybenzylamine | Me | Cl |
| 2-(aminomethyl)pyridine | Me | Cl |
| 3,4-dimethoxyphenethylamine | Me | Cl |
| 4-methoxybenzylamino | Me | Cl |
| 3-(aminomethyl) pyridine | isopropyl | Ethylenediamine |
| 4-(aminomethyl)pyridine | H | Cl |
| 6-amino-1-hexanol | H | Cl |
| phenethylamine | H | Cl |
| 2-aminobenzothiazole | H | Cl |
| 4-methoxybenzylamino | H | Cl |
| 3-phenyl-1-propylamine | isopropyl | 3-pyrroline |
| 2-aminoindane | H | Cl |
| 4-methoxyphenethylamine | H | Cl |
| 4-nitrobenzylamine | H | Cl |
| 2,6-difluorobenzytamine | H | Cl |
| 3-phenyl-1-propylamine | H | Cl |
| 2-aminoindane | Me | Cl |
| 4-methoxyphenethylamine | Me | Cl |
| 4-nitrobenzylamine | Me | Cl |
| 2,6-difluorobenzylamine | Me | Cl |
| aminomethylcyclopropane | Me | Cl |
| piperonylamine | H | Cl |
| 1-aminomethylbenzenesulfonamide | H | Cl |
| aminomethylcyclohexanol | H | Cl |
| 2-aminomethylbenzimidazole | H | Cl |
| cyclohexanmethanamine | H | Cl |
| 4-methoxybenzylamino | H | Cl |
| 4-methoxybenzylamino | isopropyl | Serinol |
| aminomethylcyclopropane | isopropyl | 1,3-Diamino-2-hydroxypropane |
| piperonylamine | Me | Cl |
| 1-aminomethylbenzenesulfonamide | Me | Cl |
| aminomethylcyclohexanol | Me | Cl |
| 2-aminomethylbenzimidazole | Me | Cl |
| cyclohexanmethanamine | Me | Cl |
| 3-(aminomethyl)pyridine | Me | Cl |
| 4-(aminomethyl)pyridine | 2-methylpropyl | Cl |
| 6-amino-1-hexanol | cyclopentyl | Cl |
| phenethylamine | propyl | Cl |
| 2-aminobenzothiazole | ethyl | Cl |
| 3-phenyl-1-propylamine | isopropyl | Cl |
| 2-aminoindane | 2-methylpropyl | Cl |
| 4-methoxyphenethylamine | cyclopentyl | Cl |
| 4-nitrobenzylamine | propyl | Cl |
| 2,6-difluorobenzylamine | ethyl | Cl |
| 4-methoxybenzylamino | isopropyl | Cl |
| Phenpropylamino | isopropyl | 4-hydroxypiperidine |
| 2-aminoindane | H | Cl |
| 2-(4-methoxyphenyl)ethylamino | H | Cl |
| 4-nitrobenzylamino | H | Cl |
| 2,6-difluorobenzylamino | H | Cl |
| 4-methoxybenzylamino | H | Cl |
| 4-methoxybenzylamino | isopropyl | 3-(Benzylamino)propionitrile |
| Phenpropylamino | isopropyl | (R/S)-Leucinol |
| 2-aminoindane | isopropyl | Cl |
| 2-(4-Methoxyphenyl)ethylamino | isopropyl | Cl |
| 4-nitrobenzylamino | isopropyl | Cl |
| 2,6-difluorobenzylamino | isopropyl | Cl |
| 4-methoxybenzylamino | isopropyl | Cl |
| 4-methoxybenzylamino | isopropyl | Piperidine |
| 4-methoxybenzylamino | isopropyl | 3-hydroxypiperidine |
| Phenpropylamino | isopropyl | L-Histidinol |
| 2-aminoindane | isopropyl | diethanolamine |
| 4-methoxybenzylamino | isopropyl | diethanolamine |
| 4-methoxybenzylamino | isopropyl | (S)-(-)-2-pyrrolidinemethanol |
| 4-methoxybenzylamino | isopropyl | Morpholine |
| 4-methoxybenzylamino | benzyl | diethanolamine |
| 4-methoxybenzylamino | 3-methylbutyl | diethanolamine |
| 4-methoxybenzylamino | 2-methylpropyl | diethanolamine |
| 4-methoxybenzylamino | cyclopentyl | diethanolamine |
| 4-methoxybenzylamino | 3-nitrobenzyl | diethanolamine |
| 4-methoxybenzylamino | 4-nitrobenzyl | diethanolamine |
| 4-methoxybenzylamino | ethyl | diethanolamine |
| 4-methoxybenzylamino | propyl | diethanolamine |
| 4-methoxybenzylamino | 3-methylbenzyl | diethanolamine |
| heptylamine | 4-methylbenzyl | diethanolamine |
| N-benzylhydroxylamine | Me | diethanolamine |
| propylamine | Me | diethanolamine |
| noradamantylamine | Me | diethanolamine |
| cyclobutylamine | Me | diethanolamine |
| 3-methoxypropylamine | Me | diethanolamine |
| 2-methoxyethylamine | Me | diethanolamine |
| cyclopentylamine | Me | diethanolamine |
| 2-amino-2-methyl-1-propanol | Me | diethanolamine |
| 4-amino-1-benzylpiperidine | Me | diethanolamine |
| 4-methoxybenzylamino | Me | diethanolamine |
| 4-methoxybenzylamino | isopropyl | 2-pyrrolidinol |
| 2,4-dimethoxybenzylamine | isopropyl | Tryptamine |
| 2-methoxybenzylamine | Me | diethanolamine |
| 2-(aminomethyl)pyridine | Me | diethanolamine |
| 3,4-dimethoxyphenethylamine | Me | diethanolamine |
| 3-(aminomethyl)pyridine | Me | diethanolamine |
| 4-(aminomethyl)pyridine | Me | diethanolamine |
| 6-amino-1-hexanol | Me | diethanolamine |
| phenethylamine | Me | diethanolamine |
| 2-aminobenzothiazole | Me | diethanolamine |
| 3-phenyl-1-propylamine | Me | diethanolamine |
| 2-aminoindane | Me | diethanolamine |
| 4-methoxyphenethylamine | Me | diethanolamine |
| 4-nitrobenzylamine | Me | diethanolamine |
| 2,6-difluorobenzylamine | Me | diethanolamine |
| aminomethylcyclopropane | Me | diethanolamine |
| piperonylamine | Me | diethanolamine |
| 1-aminomethylbenzenesulfonamide | Me | diethanolamine |
| aminomethylcyclohexanol | Me | diethanolamine |
| 2-aminoinethylbenzimidazole | Me | diethanolamine |
| cyclohexanmethanamine | Me | diethanolamine |
| 3-(aminomethyl)pyridine | Me | diethanolamine |
| 4-(aminomethyl)pyridine | 2-methylpropyl | diethanolamine |
| 6-amino-1-hexanol | cyclopentyl | diethanolamine |
| phenethylamine | propyl | diethanolamine |
| 2-aminobenzothiazole | ethyl | diethanolamine |
| 3-phenyl-1-propylamine | isopropyl | diethanolamine |
| 2-aminoindane | 2-methylpropyl | diethanolamine |
| 4-methoxyphenethylamine | cyclopentyl | diethanolamine |
| 4-nitrobenzylamine | propyl | diethanolamine |
| 2,6-difluorobenzylamine | ethyl | diethanolamine |
| 4-methoxybenzylamino | isopropyl | diethanolamine |
| 4-methoxybenzylamino | isopropyl | 1-amino-1-cyclopentanemethanol |
| 4-methoxybenzylamino | isopropyl | (+-)-2-piperidinemethanol |
| cyclopropyl | isopropyl | (+-)-3-Amino-1,2-propanediol |
| piperonylamino | isopropyl | Cl |
| 4-sulfaminobenzylamino | isopropyl | Cl |
| cyclohexanoimethylamino | isopropyl | Cl |
| 2-amino benzimidazolo | isopropyl | Cl |
| cyclohexylmethylamino | isopropyl | Cl |
| 3-phenylpropylamino | isopropyl | Cl |
| cyclopropylmethylamino | cyclopentyl | Cl |
| piperonylamino | isopropyl | diethanolamine |
| 4-methoxybenzylamino | isopropyl | diethanolamine |
| 4-methoxybenzylamino | isopropyl | Disopropylamine |
| 4-methoxybenzylamino | isopropyl | Trans-2-aminocyclohexanol |
| 4-methoxybenzylamino | isopropyl | (R)-2-Amino-3-phenyl-1-propanol |
| 4-methoxybenzylamino | isopropyl | (4S,5S)-(+)-5-amino-2,2-dimethyl-4 |
| 4-methoxybenzylamino | isopropyl | 1-(3-aminopropyl)imidazole |
| 4-methoxybenzylamino | isopropyl | 4-hydroxy-4-phenylpiperidine |
| 4-methoxybenzylamino | isopropyl | S-Benzyl-L-cysteinol |
| 4-methoxybenzylamino | isopropyl | (+-)-Epinephrine |
| 4-methoxybenzylamino | isopropyl | Diallylamine |
| 4-methoxybenzylamino | isopropyl | Piperazine |
| 4-methoxybenzylamino | isopropyl | (+-)-(Methylaminomethyl)benzylalcohol |
| 4-methoxybenzylamino | isopropyl | (S)-(+)-2-(Anilinomethyl)pyrrolidine |
| 4-methoxybenzylamino | isopropyl | 4-(Allylamino)-4-methyl-2-pentanol |
| 4-methoxybenzylamino | isopropyl | 3-(2-hydroxyethylamine)propan-1-ol |
| 4-methoxybenzylamino | isopropyl | 1,1'-dimethyl-1,1'-dipropyl-2,2'-imidodiethanol |
| 4-methoxybenzylamino | isopropyl | 3,3'-iminodi-2-butanol |
| 4-methoxybenzylamino | Me | ethanolamino |
| 4-chlorobenzyloxy | H | Cl |
| 4-chlorobenzyloxy | Me | Cl |
| 4-chlorobenzylamino | Trifluoromethyl | Cl |
| 4-methoxybenzylamino | Trifluoromethyl | Cl |
| 4-methoxybenzylamino | benzyl | Cl |
| 4-methoxybenzylamino | isopropyl | 2-aminoethylamino |
| 4-methoxybenzylamino | 2-O-TBDMS-ethyl | diethanolamino |
| 4-methoxybenzylamino | perfluoroisopropyl | Cl |
| 4-methoxybenzylamino | perfluoroisopropyl | diethanolamino |
| 4-methoxybenzylamino | 2-hydroxyethyl | diethanolamino |
| 4-methoxybenzylamino | isopropyl | 1,3-diamino-2-hydroxpropane |
| 4-methoxybenzylamino | isopropyl | N-(4-hydroxypiperidino) |
| 4-methoxybenzylamino | isopropyl | N-pyrrolidino |
| 3-phenylpropylamino | H | Cl |
| 2-aminoindanyl | H | Cl |
| 2-(4-methoxyphenyl)ethylamino | H | Cl |
| 4-nitrobenzylamino | H | Cl |
| 2,6-difluorobenzylamino | H | Cl |
| 4-methoxybenzylamino | isopropyl | N-(2-cyanopropyl)-N-(3-pyridylmethyl)-amino |
| 4-methoxybenzylamino | isopropyl | 2-(hydroxymethyl)-3-methylbutan-2-amino |
| 3-phenylpropylamino | isopropyl | Cl |
| 2-aminoindanyl | isopropyl | Cl |
| 2-(4-methoxyphenyl)ethylamino | isopropyl | Cl |
| 4-nitrobenzylamino | isopropyl | Cl |
| 2,6,difluorobenzylamino | isopropyl | Cl |
| 4-methoxybenzylamino | isopropyl | 2-(5-imidazolemethyl)ethanolamino |
| 3-phenylpropylamino | isopropyl | diethanolamino |
| 4-methoxybenzylamino | isopropyl | N-(3-hydroxpyrrolidino) |
| 4-methoxybenzylamino | isopropyl | 2-(3-indole) ethylamino |
| 4-methoxybenzylamino | isopropyl | 2,3-dihydroxypropylamino |
| 3-phenylpropylamino | cyclopentyl | Cl |
| 4-methoxybenzylamino | isopropyl | N-benzyl-N-2-hydroxyethylamino |
| 4-methoxybenzylamino | oleyl | Cl |
| 4-methoxybenzylamino | 2-naphthylmethyl | Cl |
| 4-methoxybenzylamino | 4-phenylbenzyl | Cl |
| 4-methoxybenzylamino | 1-naphthylmethyl | Cl |
| 4-methoxybenzylamino | 4-methylstilbene | Cl |
| 4-methoxybenzylamino | epoxymethyl | Cl |
| 4-methoxybenzylamino | 2,3-dihydroxpropyl | diethanolamino |
| 4-methoxybenzylamino | 4-phenylbenzyl | diethanolamino |
| 4-methoxybenzylamino | 2-phenylbenzyl | diethanolamino |
| 4-methoxybenzylamino | 2-naphthylmethyl | diethanolamino |
| 4-methoxybenzylamino | 1-naphthylmethyl | diethanolamino |
| 4-methoxybenzylamino | 4-methylstilbene | diethanolamino |
| 4-methoxybenzylamino | oleyl | diethanolamino |
| 4-phenylbenzylamino | isopropyl | 3-amino-1,2-propanediol |
| 4-phenylbenzylamino | isopropyl | hexanolamino |
| 4-phenylbenzylamino | isopropyl | bis(methoxyethyl) amino |
| 4-phenylbenzylamino | isopropyl | furfurylamino |
| 4-phenylbenzylamino | isopropyl | diethylamino |
| 4-phenylbenzylamino | isopropyl | ethanolamino |
| 4-phenylbenzylamino | isopropyl | morpholino |
| 4-phenylbenzylamino | isopropyl | 2,4-dimethoxybenzylamino |
| 4-phenylbenzylamino | isopropyl | 4-trifluoromethoxybenzylamino |
| 4-phenylbenzylamino | isopropyl | diisopropanolamino |
| 4-phenylbenzylamino | isopropyl | 2-amino-1,3-propanediol |
| 4-phenylbenzylamino | isopropyl | diallyl amino |
| 4-bromobenzylamino | isopropyl | Cl |
| 4-bromoanilino | isopropyl | Cl |
| 4-bromobenzylamino | isopropyl | diethanolamino |
| 4-bromoanilino | isopropyl | diethanolamino |
| N-methyl-4-phenylbenzylamino | isopropyl | Cl |
| 4-phenylanilino | isopropyl | diispropanolamino |
| N-methyl-4-phenylbenzylamino | isopropyl | diethanolamino |
| benzylamino | ethyl | ethanolamino |
| 4-methylbenzylamino | methyl | ethanolamino |
| 4-ethylbenzylamino | methyl | ethanolamino |
| 4-bromanlino | isopropyl | 4-bromoanilino |

### EXAMPLE 2

This Example describes a method for preparing compounds of this invention. The synthesis method disclosed in this Example is only slightly modified from that disclosed in Example 1.

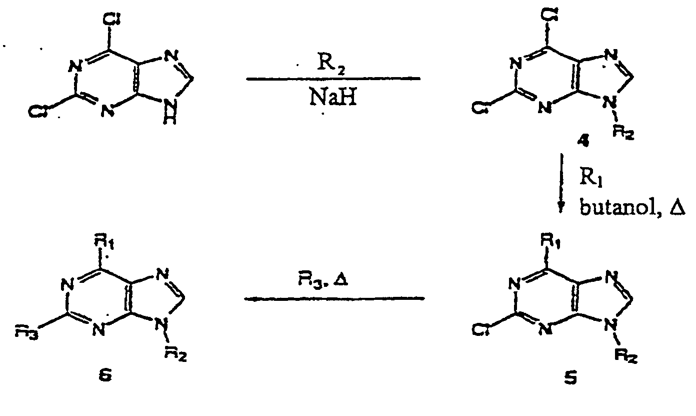

The following compound was prepared according to the method above.

### Preparation of 2,6-dichloro-9-isopropylpurine (4).

To a solution of 0.67 g of 2,6-dichloropurine in 5mL of dry DMF at room temperature was added 0. 16gms (l. 1 eq.) of 50% sodium hydride/oil powder. Upon cessation of hydrogen evolution, a large excess (2 mL) of isopropyl iodide was added to the anionic solution. This reaction solution was stirred for three days at ambient temperature. The reaction was quenched with 30 mL of water and extracted with ethyl acetate (3X50 mL). The organic extracts were combined and back washed with 3X50 mL of water followed by 20 mL of brine. The ethyl acetate solution was dried over anhydrous magnesium sulfate and evaporated. The compound was subjected to variable gradient flash chromatography on silica gel with hexane/ethyl acetate mixtures and yielded 0.37gms of desired N-9 product (45%) and 0.08gms of the N-7 isomer(10%).

### Preparation of 2-chloro-6-anilino-9-isopsopylpurine (5).

2,6-dichloro-9-isopropylpurine (0.019 g, 0.081 mmol) was dissolved in butanol (0.5 ml) and aniline (0.044 ml, 0.244 mmol) was added. The reaction mixture was heated to 120°C for 10 hr, cooled, diluted with EtOAc and washed 3 times with water. The mixture was dried over MgSO₄ and concentrated to an off white solid.

### Preparation of 2-diethanolamino-6-(4-phenylanilino)-9-isopropylpurine (6).

A solution of 67mgs of 2,6-dichloro-N-9-isopropylpurine and 100mgs of 4-phenylaniline in 1 mL of n-octanol was heated to 80°C for 24 hours. The n-octanol was removed in vacuo and then replaced with 1 mL of 40% diethanolamine in DMSO. The solution was heated at 130°C for 48 hours. The reaction was cooled to ambient temperature then diluted with 10 mL of water and subsequently extracted with ethyl acetate (3X30 mL). The organic extracts were combined and back washed with 3X20 mL of water followed by 10 mL of brine. The ethyl acetate solution was dried over anhydrous magnesium sulfate and filtered and the solvent was evaporated. The 65mgs of crude product was crystallized from THF-ether solution to yield 28mgs of pure product(23%).

Table 2 below identifies compounds of that were prepared according to the general synthesis method set forth in this Example. Those compounds, which are not encompassed by the appended claims, are given for comparative purposes only.

**TABLE 2**

| **Compounds Prepared By The Method Of Example 2** | | |
|---|---|---|
| **R1** | **R2** | **R3** |
| 8-aminoquinoline | isopropyl | Cl |
| 6-aminoquinoline | isopropyl | Cl |
| | | |
| 3-aminoquinoline | isopropyl | Cl |
| anilino | isopropyl | Cl |
| 3,5-dinitroaniline | isopropyl | Cl |
| 4-butylaniline | isopropyl | Cl |
| 8-aminoquinoline | isopropyl | diethanolamine |
| 6-aminoquinoline | isopropyl | diethanolamine |
| 3-aminoquinoline | isopropyl | diethanolamine |
| aniline | isopropyl | diethanolamine |
| 3,5-dinitroaniline | isopropyl | diethanolamine |
| 4-butylaniline | isopropyl | diethanolamine |
| 2-amino-6-ethoxybenzothiazole | isopropyl | Cl |
| 4-(2-amniomethyl)morpholine | isopropyl | Cl |
| 4-(1-aminomethyl)benzenesulfonamide | isopropyl | Cl |
| 4-bromoaniline | isopropyl | diethanolamine |
| 3,4-dichloroaniline | isopropyl | diethanolamine |
| 2-(2-aminoethyl)-1-methylpyrrolidine | isopropyl | diethanolamine |
| 3-bromoaniline | isopropyl | Cl |
| 4-anisidine | isopropyl | diethanolamine |
| 4-iodoaniline | isopropyl | Cl |
| 3-iodoaniline | isopropyl | Cl |
| m-anisidine | isopropyl | Cl |
| 1-(2-aminoethyl)piperidine | isopropyl | diethanolamine |
| 1-(2-aminoethyl)pyrrolidine | isopropyl | diethanolamine |
| 1-aminoindane | isopropyl | diethanolamine |
| 2-amino-6-ethoxybenzothiazole | isopropyl | diethanolamine |
| 4-(2-amnioethyl)morpholine | isopropyl | diethanolamine |
| 4-(1-aminomethyl)benzenesulfonamide | isopropyl | diethanolamine |
| 4-bromoaniline | isopropyl | diethanolamine |
| 3,4-dichloroaniline | isopropyl | diethanolamine |
| 2-(2-aminoethyl)-1-methylpyrrolidine | isopropyl | diethanolamine |
| | | |
| 3-bromoaniline | isopropyl | diethanolamine |
| 4-anisidine | isopropyl | diethanolamine |
| 4-iodoaniline | isopropyl | diethanolamine |
| 3-iodoaniline | isopropyl | diethanolamine |
| m-anisidine | isopropyl | diethanolamine |
| 1-(2-aminoethyl)piperidine | isopropyl | diethanolamine |
| 1-(2-aminoethyl)pyrrolidine | isopropyl | diethanolamine |
| 1-aminoindane | isopropyl | diethanolamine |
| 3-iodoaniline | isopropyl | diethanolamine |
| 3-iodoaniline | isopropyl | diethanolamine |
| 3-phenoxyaniline | isopropyl | diethanolamine |
| 4-iodoaniline | isopropyl | diethanolamine |
| 4-phenoxyaniline | isopropyl | diethanolamine |
| 3-phenoxyaniline | isopropyl | diethanolamine |
| 4-iodoanline | isopropyl | diethanolamine |
| 2-fluorenylamino | isopropyl | diethanolamine |
| 1-fluorenylamino | isopropyl | diethanolamine |
| 2-anthracenylamino | isopropyl | diethanolamine |
| 1-anthracenylamino | isopropyl | diethanolamine |
| 2-(6-ethoxybenzothiazole)amino | isopropyl | diethanolamine |
| 2-phenylbenzylamino | isopropyl | diethanolamine |
| 4-phenylbenzylamino | isopropyl | diethanolamine |
| 2-naphthylmethylamino | isopropyl | diethanolamine |
| 1-naphthylmethylamino | isopropyl | diethanolamine |

### EXAMPLE 3

This Example describes a method for preparing compounds of this invention. The synthesis method disclosed in this Example is only slightly modified from that disclosed in Example 1.

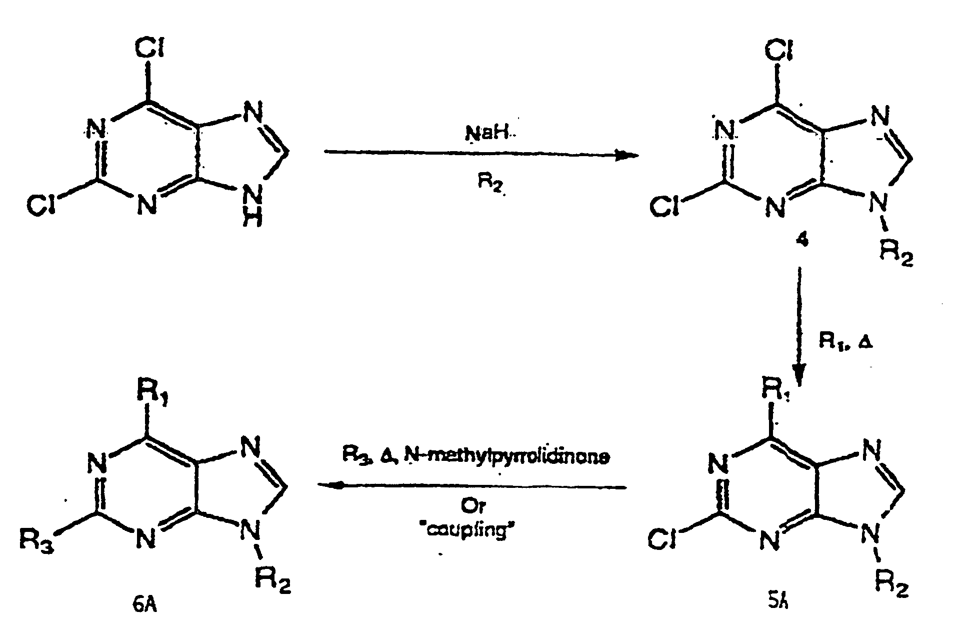

The following compound was prepared according to the method above.

### Preparation of 2,6-dichloro-9-isopropylparine (4).

The 2,6-dichloropurine (5.00 g, 26.46 mmol) was suspended in 55 ml of dry DMF at room temperature and treated with sodium hydride, 60% dispersion (1.27 g, 31.75 mmol) added in portions. After stirring for 1 hr, 2-iodopropane (4.5 ml, 44.98 mmol) was added and the reaction stirred for 2 days. The reaction was poured into diethyl ether and washed once with saturated sodium bicarbonate solution and once with water. The mixture was dried over anhydrous sodium sulfate and concentrated in vacuo. The concentrate was chromatographed over silica gel eluting with 10% acetone in dichloromethane solution to give the desired N-9 alkylation product as a white solid. Yield = 47%.

### Preparation of 2-chloro-6-(4-methylmercapto) anilino-9-isoproplypurine (5A).

2,6-Dichloro-9-isopropylpurine (0.15 g, 0.649 mmol) was dissolved in n-butanol (4 ml) and 4-(methylmercapato) aniline (0.089 ml, 0.714 mmol) and triethylamine (0.20 ml, 1.43 mmol) were added. The reaction mixture was heated at 80° overnight. The cooled reaction was diluted with ethyl acetate and washed 1 x 1M HCI, 1 x saturated sodium bicarbonate, and 1 x brine before being dried with anhydrous sodium sulfate and concentrated in vacuo. The residue was chromatographed over silica gel eluting with 2% methanol in dichloromethane to give the desired product as a white solid. Yield = 83%.

### Preparation of 2-diethanolamine-6-(4-methylmercapto) anilino-9-isopropylpurine (6A).

The purine (0.18 g, 539 mmol) was dissolved in N-methylyrrolidinone (3 ml) and diethanolamine (1 ml) and then heated at 120°C overnight. The cooled reaction was poured into diethyl ether and washed three times with water before drying over anhydrous sodium sulfate and concentrating in vacuo. The residue was chromatographed over silica gel eluting with 5% methanol in dichloromethane to give the desired product as an off-white solid. Yield = 82 %. ¹H-NMR(δ, CDCl ₃) : 8.08(s,1H), 7.58(d, 2H), 7.47(s,1H), 7.18(d, 2H), 4.95(br s, <2H), 4.52(m, 1H), 3.94(m, 4H), 3.83(m,4H), 2.43(s, 3H), 1.47(d, 6H).

### Preparation of 4-(2-thienyl) benzonitrile.

Some R₁' groups must first be synthesized before reacting with the 2,6-dichloro-9-isopropylpurine. These groups can be synthesized through various coupling methods and other synthetic procedures known to those skilled in the art of organic synthesis.

To a pressure tube was added 4-bromobenzonitrile (0.20 g, 1.10 mmol), tetrakis(triphenylphosphine) palladium (0) (0.127 g, 0.1 eq) and 2-thiopheneboronic acid (0.211 g, 1.65 mmol). The reaction was flushed under vacuum and flushed with dry nitrogen three times. Following flushes, ethyleneglycol dimethyl ether (5.5 ml) and an aqueous solution of sodium carbonate (2.53 ml, 1M) were added to the tube. The tube was then sealed and heated at 80°C overnight. The cooled reaction was diluted with diethyl ether and washed twice with water before drying over sodium sulfate and concentrating in vacuo. The residue was chromatographed over silica gel eluting with 10% ethyl acetate in hexane to give the desired product as a white solid. Yield = 84%.

### Preparation of 4-(2-thienyl) benzylamine.

The 4-(2-thienyl)benzonitrile (0.086 g, 0.464 mmol) was dissolved in dry tetrahydrofuran (1.6 ml) before lithium aluminum hydride (0.46 ml, 0.464 mmol, 1 M in THF) was added dropwise. The reaction was allowed to stir at room temperature overnight. TLC (5% methanol in dichloromethane) still showed starting material remaining. Another 1 eq of LAH was added. After an additional hour, the reaction was quenched by the Fieser and Fieser method using wager (17.46µl), aqueous sodium hydroxide solution (17.46µl, 15% soln.), and water (52.37 µl) added sequentially to the reaction. The reaction was then diluted with diethyl ether and water and extracted twice with diethyl ether before drying over sodium sulfate and concentrating in vacuo. The residue was carried on crude without any further purification. Yield = 89%.

Table 3 below identified compounds that were prepared according to the general synthesis method set forth in this Example. Those compounds, which are not encompassed by the appended claims, are given for comparative purposes only.

**Table 3**

| **Compounds Prepared By The Method of Example 3** | | |
|---|---|---|
| **R**_{**1**} | **R2** | **R3** |
| Cl | Me | Cl |
| 3,5-dinitroanilino | isopropyl | Cl |
| 3-phenoxyanilino | isopropyl | Cl |
| 4-iodoanilino | isopropyl | Cl |
| 3-aminoquinolino | isopropyl | Cl |
| 3,5-dinitroanilino | isopropyl | diethanolamino |
| Cl | epoxymethyl | Cl |
| 4-methoxybenzylamino | 2,3-dihydroxypropyl | diethanolamino |
| 4-phenylanilino | isopropyl | diethanolamino |
| 4-phenylbenzylamino | isopropyl | Cl |
| 2-naphthalenylmethylamino | isopropyl | Cl |
| 1-naphthalenylmethylamino | isopropyl | Cl |
| 2-phenylbenzylamino | isopropyl | Cl |
| 3-quinolinylamino | isopropyl | diethanolamino |
| 5-quinolinylamino | isopropyl | diethanolamino |
| 6-quinolinylamino | isopropyl | diethanolamino |
| 8-quinolinylamino | isopropyl | diethanolamino |
| n-butylamino | isopropyl | Cl |
| 4-(2-thiophenyl)benzylamino | isopropyl | diethanolamino |
| 4-(2-thiophenyl)benzylamino | isopropyl | Cl |
| 3-thiomethoxyanilino | isopropyl | Cl |
| 4-thiomethoxyanilino | isopropyl | Cl |
| 3-thiomethorxyanilino | isopropyl | diethanoamino |
| 4-thiomethoxyanilino | isopropyl | diethanoamino |
| 4-(2-pyridinyl)benzylamino | isopropyl | Cl |
| 3-methoxybenzylamino | isopropyl | Cl |
| 3,4-dimethoxybenzylamino | isopropyl | Cl |
| 3,4,5-trimethoxyenzylamino | isopropyl | Cl |
| 3-methoxybenzylamino | isopropyl | diethanolamino |
| 3,4-dimethoxybenzylamino | isopropyl | diethanolamino |
| 3,4,5-trimethoxbenzylamino | isopropyl | diethanolamino |
| 4-(3-thiophenyl)benzylamino | isopropyl | Cl |
| 4-(4-methoxphenyl)benzylamino | isopropyl | Cl |
| 4-(4-bromophenyl)benzylamino | isopropyl | diethanolamino |
| 4-(3- methoxyphenyl)benzylamino | isopropyl | diethanolamino |
| 4-(4-methoxypheny)benzylamino | isopropyl | diethanolamino |
| 4-(3-thiophenyl)benzylamino | isopropyl | diethanolamino |
| 4-(3-methylpheny)benzylamino | on isopropyl | Cl |
| 4-(4-methylphenyl)benzylamino | isopropyl | Cl |
| 4-(4-trifluoromethylphenyl)benzylamino | isopropyl | Cl |
| 3-(4 nitrilophenyl)anilino | isopropyl | Cl |
| 3-(4-nitrilophenyl)anilino | isopropyl | diethanolamino |
| 4-(2-pyridinyl)benzylamino | isopropyl | Cl |
| 4-(2-pryidinyl)benzylamino | isopropyl | diethanolamino |

### EXAMPLE 4

This Example describes a method for preparing compounds of this invention. The synthesis method disclosed in this Example is only slightly modified from that disclosed in Example 1.

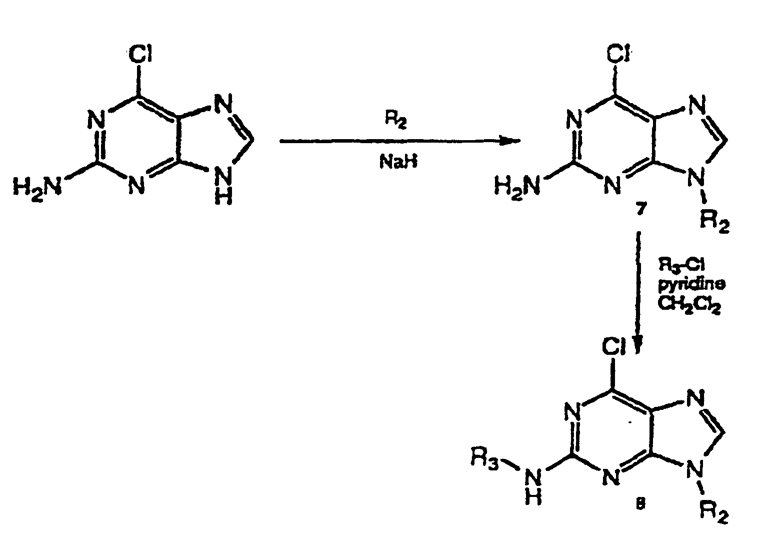

The following compound was prepared according to the method above.

### Preparation of 2-amino-6-chloro-9-methylpurine (7).

The 2-amino-6-chloropurine (1.08 g, 6.4 mmol) was suspended in dry DMF (75 ml) and treated with sodium hydride, 60% dispersion (0.28 g, 7 mmol). The suspension was stirred for 15 min before iodomethane (0.44 ml, 7.06 mmol) was added and the resulting yellow solution stirred for 1 hr 45 min. The solid was filtered and the filtrate evaporated before addition of water for 10 min. The resulting solid was filtered and dried overnight to give the product as a mixture of N-7 and N-9 alkylation products. The residual liquor was left overnight and more crystals were collected the next day and dried. Yield = 77%.

### Preparation of 6-chloro-2-(2-methoxyacetylamino)-9-methylpurine (8).

The mixture of isomers from above was dissolved in dichloromethane and pyridine (2 eq) followed by treatment with methoxyacetyl chloride (4 eq). The reaction was stirred at room temperature until complete. The reaction was evaporated and filtered through a plug of silicia gel eluting with 2% methanol in dichloromethane followed by purification on a chomatotron using silica gel and eluting with 2% methanol in dichloromethane to isolate the desired product. Yield = 31%.

Table 4 identifies compounds that were prepared according to the synthesis method set forth in this Example.

**Table 4**

| **Compounds Prepared By The Method of Example 4** | | |
|---|---|---|
| **R1** | **R2** | **R3** |
| Cl | Me | H |
| Cl | Me | 2-methoxyacetylamino |

### EXAMPLE 5

This Example describes a method for preparing compounds of this invention. The synthesis method disclosed in this Example is only slightly modified from that disclosed in Example 1.

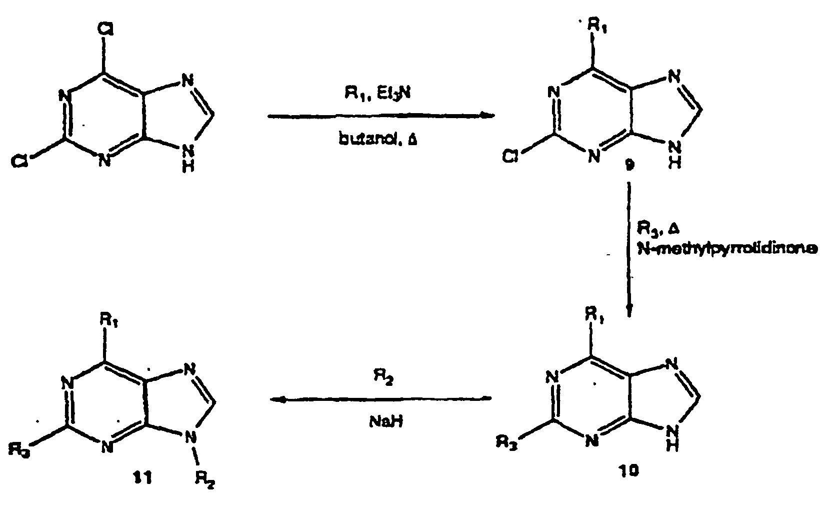

The following compound was prepared according to the method above.

### Preparation of 2-chloro-6-(4-phenyl benzylamino) purine (9).

The 2,6-dichloropurine (5.0 g, 26.45 mmol) was suspended in n-butanol (50 ml) and the 4-phenylbenzylamine (6.61 g, 29.1 mmol) and triethylamine (4.1 ml, 29.1 mmol) were added. The solution was heated at 120°C overnight then cooled. Filtered off product using excess n-butanol and washed precipitate with 100 ml 1M HCl and 200 ml water. The solid was dried in vaccum overnight at 70°C to give the desired product as a pale yellow solid. Yield = 99%.

### Preparation of 2-diethanolamino-6-(4-phenyl benzylamino) purine (10).

The 2-chloro-6-(4-phenyl benzylamino) purine (2.0 g, 5.96 mmol) was added together with diethanolamine (11.4 ml, 119.2 mmol) and N-methylpyrrolidinone (10 ml) and heated at 120°C overnight..The cooled reaction was poured into dichloromethane and washed twice with water. The organic layer was dried with anhydrous sodium sulfate and concentrated in vacuo to give the desired product as a pale green solid which was further dried in vacuum oven at 70°C for 2 days.

### Preparation of 2-diethanolamino-6-(4-phenyl benzylamino)-9-methylpurine (11).

The 2-diethanolamino-6-(4-phenyl benxylamino) purine (0.050 g, 0.124 mmol) was dissolved in dry DMF and treated wit sodium hydride, 60% dispersion (5.5 mgs, 0.136 mmol) for 1 hr. iodomethane (0.009 ml, 0.148 mmol) was added and the resultant solution stirred at room temperature overnight. Poured reaction into diethyl ether and washed twice with saturated sodium bicarbonate solution before drying over anhydrous sodium sulfate and concentrating in vacuo. The residue was chromatographed over silica gel eluting with 5% methanol in dichloromethane to give the produce as a white solid. Yield = 63%.
₁H-NMR(δ, CDCl3): 7.55 (m,4H), 7.41 (m, 4H) 7.35(m, 4H), 6.41 (br s, < 1H), 5.10(br s, <2H), 4.72 (br s, 2H); 3.86 (m, 4H), 3.74(m, 4H), 3.59(s, 3H).

Table 5 identified compounds that were prepared according to the synthesis method set forth in this Example. Those compounds, which are not encompassed by the appended claims, are given for comparative purposes only.

**Table 5**

| **Compounds Prepared By The Method of Example 5** | | |
|---|---|---|
| **R1** | **R2** | **R3** |
| 4-phenylbenzylamino | methyl | diethanolamino |
| 4-phenylbenzylamino | cyclopentyl | diethanolamino |
| 4-phenylbenzylamino | allyl | diethanolamino |
| 4-phenylbenzylamino | benzyl | diethanolamino |
| 4-phenylbenzylamino | 3-methylbutyl | diethanolamino |
| 4-phenylbenzylamino | isobutyl | diethanolamino |
| 4-phenylbenzylamino | t-butylacetate | diethanolamino |
| 4-phenylbenzylamino | methylacetate | diethanolamino |
| 4-phenylbenzylamino | cyclobutyl | diethanolamino |
| 4-phenylbenzylamino | ethyl | diethanolamino |
| 4-phenylbenzylamino | propyl | diethanolamino |

### EXAMPLES 6

Compounds of this invention were evaluated in the following assays.

### CDK2 assays:

Compounds of this invention were assayed to determine their CDK2 inhibitory activity. The assay system (total volume of 50 µl) contained 50 mM Tris-Cl, pH 7.4, 10 mM MgCl₂, 5 mM DTT, 1 µg of histone H1, 30 µM ATP (1 µCl of gamma³²P labeled ATP), 10 µg or BSA and 1 ng of purified CDK2. After incubation at 30°C for 30 min, the reaction was terminated by the addition of 10 µl of 10% TCA and the samples were blotted onto to nitrocellulose filters. These filters were washed extensively in 10% TCA and assayed for raoloacdvity. Blanks contained no enzyme. To ascertain the potency of various compounds of this invention, the compounds were added to the above assay at concentrations ranging from 100 to 0.02 µg/ml. After incubation at 30 min., the assay tubes were processed as above. In all assays, various concentrations of olomoucine were added and were used as a standard positive control. The IC₅₀ (enzyme) listed in Table 6 is defined as the concentration required to inhibit CDK2. activity by 50%.

### EXAMPLE 7

### Cell Proliferation Assays:

Early passage rat aortic smooth muscle cells (CV Therapeutics Cell repository) were seeded in 48 well dishes (Falcon, ml/well) at a density of 20,000 cells/ml of DME containing 5% heat inactivated bovine serum. The cells were incubated in a standard tissue culture incubator for 48 hr. The medium was aspirated and the wells were replenished with 0.2 ml of fresh medium. Compounds of this invention were added at concentrations ranging from 100 to 0.37 µg/ml. After 48 hr incubation, the medium was aspirated and the cultures were treated with 0.2 ml of saline 0.25 µl of phenozine methosulfate solution containing MTS (Cell Titer 96® Aqueous Non-radioactive cell proliferation assay kit, Catalog # G 5430, Promega, 2800 Woods Hollow Road, Madison, WI 53711-5399). The IC₅₀ cells listed in Table 6 is defined as the concentration required to inhibit cell proliferation by 50%. Olomoucine at various concentrations was added and was used as a standard positive control. Table 6 indicates the bioactivity of selected representatives of the invention, wherein those compounds, which are not encompassed by the appended claims, were tested for comparative purposes.

**TABLE 6**

| **R1** | **R2** | **R3** | **IC**_{**50**} **(µg/mL) enzyme** | **IC**_{**50**} **(µg/mL) cells** |
|---|---|---|---|---|
| 4-methoxybenzylamino | H | Cl | 60 | NA |
| 4-methoxybenzylamino | Me | Cl | 6 | >70 |
| 4-methoxybenzylamino | Me | ethanolamino | 4 | 48 |
| 4-chlorobenzyloxy | H | Cl | 60 | NA |
| 4-chlorobenzyloxy | Me | Cl | 60 | NA |
| 4-chlorobenzyloxy | trifluoromethyl | Cl | >60 | NA |
| 4-methoxybenzylamino | isopropyl | Cl | 4 | 77 |
| 4-methoxybenzylamino | isopropyl | ethanolamino | 4 | 43 |
| 4-methoxybenzylamino | Me | diethanolamino | 4 | 48 |
| 4-methoxybenzylamino | 2-methylpropyl | Cl | 60 | >70 |
| ethanolamino | Me | ethanolamino | >60 | >70 |
| 4-methoxybenzylamino | trifluoromethyl | Cl | >60 | >70 |
| 4-methoxybenzylamino | benzyl | Cl | >60 | >70 |
| ethanolamino | H | benzylamino | >60 | NA |
| 4-methoxybenzylamino | isopropyl | diethanolamino | 0.2 | 2.1 |
| 4-methoxybenzylamino | perfluoroisopropyl | Cl | >45 | NA |
| 4-methoxybenzylamino | perfluoroisopropyl | diethanolammo | 40 | NA |
| 4-mechoxybenzylamino | ispropyl | 3-pyrroline | 1 | 12.5 |
| 4-methoxybenzylamino | hydroxyethyl | diethanolamino | 0.5 | 62 |
| 4-methoxybenzylamino | isopropyl | serinol | 0.4 | 15 |
| 4-methoxybenzylamino | isopropyl | 1,3-diamino-2-hydroxypropane | 0.6 | 25 |
| 4-methoxybenzylamino | 3-cyanopropyl | Cl | >60 | NA |
| 4-methoxybenzylamino | 3-chloropropyl | Cl | >60 | NA |
| 4-methoxybenzylamino | benzyl | Cl | >60 | NA |
| 4-methoxybenzylamino | Methyl 4-carboxybenzyl | Cl | >60 | NA |
| 4-methoxybenzylamino | Naphthaloylethyl | Cl | >60 | NA |
| 4-chlorobenzylamino | Trifluoromethyl | Cl | 1 | NA |
| 4-methoxybenzylamino | isopropyl | N-(2-cyanopropyl)-N-(3-pyridylmethyl)-amino | 1 | NA |
| 4-methoxybenzylamino | isopropyl | 2-(hydroxymethyl)-3-methylbutan-Z-amino | 1 | NA |
| 4-methoxybenzylamino | isopropyl | 3-hydroxypiperidino | 1 | NA |
| cyclohexylmethylamino | isopropyl | Cl | 1 | NA |
| piperonylamino | isopropyl | diethanolamino | 0.8 | NA |
| 4-methoxybenzylamino | isopropyl | diisopropanolamino | 0.8 | NA |
| anilino | isopropyl | Cl | 1 | NA |
| 4-methoxybenzylamino | isopropyl | N-benzyl-N-2-hydroxyethylamino | 1 | NA |
| 4-phenylanilino | isopropyl | diethanolamino | 0.6 | NA |
| 4-phenylbenzylamino | isopropyl | diethanolamino | 0.6 | NA |
| 4-phenylbenzylamino | isopropyl | 3-amino-1,2-propanediol | 0.6 | NA |
| 4-(2-thiophenyl)benzylamino | isopropyl | diethannolamino | 0.5 | NA |
| 4-(4-methylphenyl)benzylamino | isopropyl | diethanolamino | 0.6 | NA |
| 4-(4-trifluoromethylphenyl)benzyalmino | isopropyl | diethanolamino | 0.6 | NA |
| 4-thiomethoxyanilino | isopropyl | Cl | 0.6 | NA |
| 3-(4-nitrilophenyl)anilino | isopropyl | diethanolamino | 0.5 | NA |
| 3-thiomethoxyanilino | isopropyl | diethanolamino | 0.1 | NA |
| 4-thiomethoxyanilino | isopropyl | diethanotamino | 0.07 | NA |
| 3-methoxybenzylamino | isopropyl | Cl | 0.9 | NA |
| 4-(2-pyridinyl)benzylamino | isopropyl | diethanolamino | 0.16 | NA |
| 3-methoxybenzylamino | isopropyl | diethanolamino | 0.5 | NA |

The inhibition of cell proliferation properties of the compounds of this invention are demonstrated by their ability to inhibit cell proliferation in the range of about 0.05 µg/ml to 100 µg/ml, preferably less than 0.5 µg/ml.

### EXAMPLE 7

A compound of this invention was evaluated for effectiveness using the Murine Leukemia Model. The Murine Leukemia Model is a standard model used in the evaluation of antitumor agents. CDF1 mice were injected ip with L1210 cells (1x10³ cells/mouse). Twenty-four hours later, these mice were treated with various doses (ip) of compound 3 of Example 1 in saline. The dosing regimen used in this study is outlined in Table 7, below. Mice were dosed with compound 3 daily or on alternate days. Control mice received saline. After 7 days, dosing was suspended and survival monitored.

**Table 7**

| **Treatment** | | **N** | **Median survival time Days** | **T/Cx100** |
|---|---|---|---|---|
| Saline control | | 7 | 10 (9-13) | 100 |
| | | | | |
| Compound 3 | 0.5 mg/kg bid | 7 | 11 (10-15) | 110 |
| | 1.0 mg/kg bid | 7 | 13 (11-13) | 130 |
| | 2 mglkg bid | 7 | 12 (10-14) | 120 |
| | 4 mg/kg - days 1,3,5,7 | 7 | 13 (10-15) | 130 |
| | 8 mg/kg - days 1,3,5,7 | 7 | 13 (12-16) | 130 |

The results indicate that rats administered compound 3 survived longer than the control rats.

### EXAMPLE 8

This example measured the effect of an acute local delivery of compound 3 of Example 1 in reducing neointima formation following balloon angioplasty in the rat carotid artery model. In this example, the left common carotid arteries of adult male rats (n=10 per experimental group) were surgically injured using a Fogarty arterial embolectomy catheter. Immediately after injury, the common carotid artery was bisected with a vascular clamp, thereby establishing an untreated and treated segment. A drug delivery catheter was then inserted into the distal half of the common carotid. After drug delivery, the catheter was removed and excess drug was washed out by removing the vascular clamp and re-establishing blood flow before closing the artery. The animals were allowed to recover for 14 days before harvesting the common carotid artery. The harvested tissue was sectioned and the neointimal area was digitized and measured with a computer planimetery system. For each animal, 15 measurements were averaged for the untreated segment and 15 for the treated.

The results of this Example are found in Figure 1. According to Figure 1, administering compound 3 of Example 1 to a damaged carotid artery reduced the neointimal area about 88% in comparison to the 6% reduction produced by the saline vehicle alone.

### EXAMPLE 9

### IκB-α Kinase Assays:

Compounds of this invention were assayed to determine their IκB-α kinase inhibitory activity. The human umbilical vein endothelial cell line (HUVEC) used in these studies was purchased from Clonetics (San Diego, CA) and was maintained in endothelial cell growth medium supplemented with 2% fetal bovine serum, 10ng/ml human recombinant epidermal growth factor, 1 µg/ml hydrocortisone, 50 µg/ml gentamicin, 50 ng/ml amphotericin B and 12 µg/ml bovine brain extract at 37°C in a tissue culture incubator. All growth media and supplements were purchased from Clonetics (San Diego, CA). *E. coli* lipopolysaccharide (LPS) serotype 0111:B4 was purchased from Sigma (Saint Louis, MI). All other chemicals were of reagent grade.

Preparation of cell Lysate: Monolayers (75 cm²) of HUVEC cells were treated with LPS (100 ng/ml) for 5 minutes after which the cell media was rapidly removed and the monolayer washed three times with ice cold PBS. The cell layer was scraped into 10 ml PBS and the cells pelleted by centrifugation (3000 rpm, 5 min, 4°C). Cell lysate was prepared by incubating the cell pellet in 0.2 ml lysis buffer (20mM HEPES, pH7.3, 50mM NaCl, 10mM MgCl₂, 1mM EDTA, 1mM EGTA, 1mM sodium orthovanadate, 10mM β-glycerophospate, 1mM phenylmethylsulfonylfuoride, 1mM dithiothreitol, 0.5% Nonidet P-40 for 15 minutes at 37°C for frequent vortexing. Cell debris was removed from the sample by microcentrifugation (10,000xg, 15 minutes, 4°C) and the supernatant was "precleared" by the addition of 100 ml of a suspension of sepharose 4B in lysis buffer and mixing gently for 1 hour at 4°C. The speharose 4B beads were removed by microcentrifugation and the supernatant aliquotted and stored at 80°C.

Solid Phase IκB-α kinase assay**:** 1 µg of GST- IκB-α, corresponding to full length IκB-α of human origin, (Santa Cruz Biotechnology,) was incubated with 20 µl of a 50% slurry of glutathione S sepharose 4B (Pharmacia) in reaction buffer (20mM HEPES, pH7.3, 10mM MgCl₂, 15mM β-glycerophosphate, 0.5mM sodium orthovanadate, 0.5mM EGTA) for 30 minutes at room temperature. The GST- IκB-bead complex was washed three times with 0.5 ml of reaction buffer by resuspension and microcentrifugation. 10µg of HUVEC cell lysate protein in 100µl of reaction buffer was then added to the GST- IκB-bead complex and the mixture incubated with gentle mixing at 4°C for 1 hour. The bead complex was then washed three times with reaction buffer containing 0.2 M NaCl and once with reaction buffer alone. Finally the bead complex was resuspended in 20µl of reaction buffer containing 5µCi [y-³²P]ATP (>5000 ci/mmol, New England Nuclear Corp. Boston, MA) and incubated at room temperature for 15 minutes. The reaction was terminated by the addition of 10µl of SDS-PAGE sample buffer and boiled for 3 minutes before separation by SDS-PAGE (10-20% gradient Readygel, BioRad): Following electrophoresis the gel was fixed (50% methanol 10% acetic acid) for 15 minutes, washed three times for 5 minutes each with distilled H₂O and treated with 5% glycerol for 15 minutes before drying down and exposing to film for autoradiography (X-OMAT XAR-5 Kodak).

In gel kinase assay: IκB-α isozymes were assayed for activity using a modification of previously published methods (11, 19, 20). Briefly duplicate samples of the IκB-glutathione sepharose 4B bead complex were prepared as described above and were separated by electrophoresis through a 12% SDS-PAGE gel which had been polymerised in the presence of 15 µg/ml GST- IκB-α. Following electrophoresis the gel was washed gently twice for 30 minutes each with 50mM Tris-HCI pH8.0, 5mM β-mercaptoethanol; 20% isopropanol to remove SDS. Proteins were then denatured within the gel by incubation for 45 minutes in 100ml 50mM Tris-HCl pH8.0; 5mM β-mercaptoethanol; 0.04% Tween 40. The gel was then cut in half to separate the duplicate samples, one half was incubated in 10 ml reaction buffer alone and the other in 10 ml reaction buffer containing 10µg/ml of 2-diethanolamino-6(4-phenyl anilino)-9-isopropyl purine (compound 6 of Example 2) for 1 hour at room temperature which 10µCi[y-³² P]ATP was added and the incubations continued for a further hour at room temperature. The gels were then subjected to multiple 15 minute washes of 100ml each 5% trichloroacetic acid containing 1% sodium pyrophosphate until 1 ml of wash solution gave close to background radioactivity. The gels were then dried down and exposed to file for autoradiograhy.

Preparation of 2-diethanolamino-6-(4-phenylbenzylamino)-9-isopropyl purine Epoxy activated Sepharose 6B Affinity Matrix. Freeze dried epoxy activated Sepharose 6B (Pharmacia LKB, Piscataway, NJ) was chosen for the coupling reaction due to its ability to form an ether bond between an hydroxyl-containing ligand and the epoxide group on the sepharose. The gel was swollen according to the manufacturer's instructions, (100mg) of compound 6 of Example 2. was dissolved in 1ml coupling solution (1.2:1 v/v dimethylformamide : 0.1N NaOH) and mixed with 0.5ml of swollen gel at pH 10-11 for 72 hours at room temperature with gentle agitation. Excess reactive groups were blocked with 1M ethanolamine for 4 hours at 50°C and the gel slurry was poured into 1 ml syringe column. The resin was activated with three alternating cycles of twenty column volumes each of pH 4.0 (0.1M acetate, 0.5M NaCl) and pH 8.0 (0.1M Tris-HCl, 0.5M NaCl) buffers followed by twenty column volumes of reaction buffer (20mM HEPES, pH7.3, 10mM MgCl₂, 15niM β-glycerophophate, 0.5mM sodium orthovanadate, 0.5mM EGTA). The column was stored at 4°C in reaction buffer containing 0.5% sodium azide and regenerated prior to each use with alternating cycles of low and high pH as described above.

Activated HWEC cell lysate (500µg protein in 1ml reaction buffer) was passed over the CVT-1545 sepharose matrix sequentially five times and the flow through was saved (unbound material). The matrix was then washed three times with 1ml of reaction buffer (wash 1-3) then three times each with reaction buffer containing 0.5M NaCl (eluate 1-3). Aliquots (20µl from 1ml) of each sample were assayed for their ability to phosphorylate at GST- IκB-sepharose bead complex and analyzed by SDS-PAGE as described above.

Assay of affinity enriched IκB-α kinase. The bulked 0.5 M NaCl eluates from the affinity matrix were used as the source of enzyme for development of an IκB-α kinase filter assay. Each reaction contained affinity enriched IκB-α kinase (1µg protein), 10ng GST IκB-α kinase and 0.5µCi[y-³²P]ATP (>5000 Ci/mmol, New England Nuclear Corp, Boston, MA) in 20µl reaction buffer. The reaction was incubated for 15 minutes at room temperature and was terminated by the addition of 2µl 0.5M EDTA. Reaction mixtures were blotted onto phosphocellulose disks (Gibco BRL Life Technologies, Gaithersburg, MD) and the filters washed three times with 0.15M phosphoric acid with gentle shaking for 15 minutes (up to ten filters were washed with 300 ml of 0.15M phosphoric acid.) Following a third wash the filters were air dried, added to scintillation fluid and assayed by liquid scintillation spectrometry.

Electrophoretic Mobility Shift Assay: Nuclear extracts were prepared using a high-salt buffer extraction procedure. 10 pmol of double stranded NF-κB consenses oligonucleotide (5'-AGTTGAGGGGACTTTCCCAGGC-3'))Promega) was 5' end labeled with 5µCi [y-³²P]ATP (>5000 Ci/mmol, New England Nuclear Corp, Boston, MA) by incubaton with T4 polynucleotide kinase for 1 hr at 37°C. Unincorporated nucleotides were removed by pasing the reaction mixture over 1ml Sephadex G-5-spin column. Binding assays were performed at room temperature for 1 hr and consisted of 10µg nuclear extract protein, 1µg salmon sperm DNA, and 5x10⁴ cpm of ³²P labeled consensus of oligonucleotide in the presence and absence of fifty fold unlabeled oligonucleotide. DNA-protein complexes were resolved by 8% non denaturing polyacrylamide gel electrophoresis, the gels were dried onto filter paper and visualized by autoradiography. Table 8 indicates the enzyme activity of selected representatives of this invention, wherein those compounds, which are not encompassed by the appended claims were tested for comparative purposes.

**Table 8**

| **R1** | **R2** | **R3** | **IC50(µM) enzyme** |
|---|---|---|---|
| 4-phenylbenzylamino | isopropyl | diethanolamino | 1.1 |
| 4-phenylbenzylamino | isopropyl | diethylamino | >2.4 |
| 4-phenylbenzylamino | isopropyl | ethanolamino | 2.5 |
| 4-bromoanilino | isopropyl | diethanolamino | 14 |
| 4-(3-methoxphenyl) benzylamino | isopropyl | diethanolamino | >10 |
| 4-(4-methoxphenyl) benzylamino | isopropyl | diethanolamino | 11 |
| 3-(4-nitrilophenyl) anilino | isopropyl | diethanolamino | 2.2 |
| 4-thiomethoxyanilino | isopropyl | diethanolamino | 12.4 |
| 4-(2-pyridinyl) benzylamino | isopropyl | diethanolamino | 4.5 |

## Claims

1. A compound having the general formula: 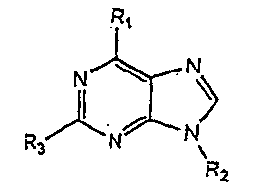 or a pharmaceutically acceptable salt thereof wherein
R₁ is halogen;
R₂ is C₂ to C₁₀ alkyl optionally substituted with one, two or three groups selected from hydroxy, halogen, and -C(O)R;
R and R' independently represent hydrogen, or
lower alkyl optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
aryl or heteroaryl optionally substituted with one, two or three groups selected from lower alkyl, alkoxy, halogen, mercapto, alkylthio, acetylene, amino, mono- or dialkylamino, -C(O)NH-alkyl, -C(O)N-dialkyl, -C(O)O-alkyl, -C(O)alkyl, hydroxy, aryl, aryloxy, heteroaryl, nitro or cyano; and
R₃ is halogen, -NR₄R₅ or a moiety having the general formula: 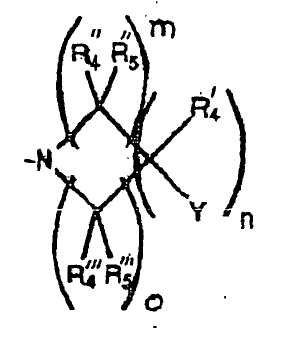
where
m, n, and o independently represent 1, 2 or 3;
Y is -NR₄R₅, hydroxy, mercapto, -OR, alkylthiol; and
R₄, R₄', R₄", R₄''', R₅, R₅" and R₅''' are each independently hydrogen, hydroxy,
lower alkyl, optionally substituted with one, two or three groups selected from hydroxy, NRR', or
aryl, heteroaryl, or arylalkyl; or
Y and R₄' together may be a single oxygen atom; or
R₄" and R₅" together may be a single oxgen atom; or
R₄''' and R₅''' together may be a single oxygen atom.

2. A compound according to claim 1 wherein R₁ is chloro.

3. A compound according to claim 2 wherein R₂ is C₂ to C₁₀ alkyl optionally substituted with one, two or three hydroxy groups.

4. A compound according to claim 2 wherein R₃ is halogen or -NR₄R₅.

5. A compound according to claim 4 wherein R₃ is chloro.

6. A compound according to claim 4 wherein R₃ is -NR₄R₅.

7. A compound having the general formula 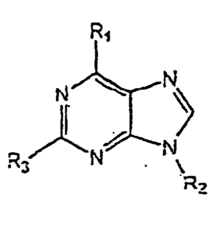 or a pharmaceutically acceptable salt thereof wherein
R₁ is -NHR₁';
R₁' is quinolin-3-yl or quinolin-6-yl; or
R₁' is benzyl where the ring portion is substituted with one, two or three groups selected from halogen, -OR, thienyl, nitro, pyridyl, piperonyl, or
phenyl, where the phenyl is unsubstituted or substituted with trifluoromethyl, halogen,
lower alkyl, lower alkoxy, hydroxy, amino, mercapto, alkylthio, nitro or cyano; or
R₁' is phenyl substituted with one, two or three groups selected from bromo, iodo, -OR", thienyl, pyridyl, piperonyl, or
phenyl, where the phenyl is unsubstituted or substituted with trifluoromethyl, halogen,
lower alkyl, lower alkoxy, hydroxy, amino, mercapto, alkylthio, nitro or cyano
R₂ is C₂ to C₁₀ alkyl optionally substituted with one, two or three groups selected from hydroxy, halogen, and -C(O)R;
R and R' independently represent hydrogen, or
lower alkyl, optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
aryl or heteroaryl optionally substituted with one, two or three groups selected from lower alkyl, alkoxy, halogen, mercapto, alkylthiol, acetylene, amino, mono- or dialkylamino, -C(O)NH-alkyl, -C(O)N-dialkyl, -C(O)O-alkyl, - C(O)alkyl, hydroxy, aryl, aryloxy, heteroaryl, nitro or cyano;
R" represents hydrogen, or
C₂ to C₁₀ alkyl, optionally substituted with one, two or three groups selected from hydroxy, -O-alkyl, halogen, amino, mono- or dialkylamino, mercapto, alkylthiol, -C(O)NH-alkyl, -C(O)N-dialkyl, -C(O)O-alkyl, cyano, aryloxy, alkenyl, alkynyl, -C(O)alkyl, or
aryl or heteroaryl optionally substituted with one, two or three groups selected from lower alkyl, alkoxy, halogen, mercapto, alkylthiol, acetylene, amino, mono- or dialkylamino, -C(O)NH-alkyl, -C(O)N-dialkyl, -C(O)O-alkyl, - C(O)alkyl, hydroxy, aryl, aryloxy, heteroaryl, nitro or cyano;
R₃ is halogen, -NR₄R₅ or a moiety having the general formula: 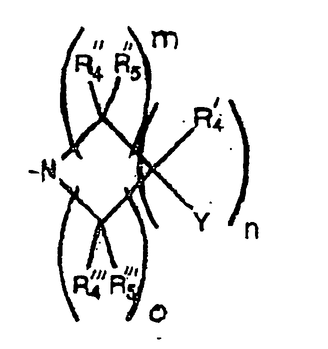
where
m, n, and o independently represent 1, 2 or 3;
Y is -NR₄R₅, hydroxy, mercapto, -OR, alkylthio ; and
R₄, R₄', R₄", R₄''', R₅, R₅" and R₅''' independently are hydrogen, hydroxy,
lower alkyl optionally substituted with one, two or three groups selected from hydroxy, -NRR', or
aryl, heteroaryl, or arylalkyl; or
Y and R₄' together may be a single oxygen atom; or
R₄" and R₅" together may be a single oxgen atom; or
R₄''' and R₅''' together may be a single oxygen atom.

8. A compound according to claim 7
wherein R₁ is NHAr; and
Ar is phenyl substituted with one, two or three groups selected from bromo, iodo -OR", thienyl, pyridyl, piperonyl, or
phenyl, where the phenyl is unsubstituted or substituted with trifluoromethyl, halogen, lower alkyl, lower alkoxy, hydroxy, amino, mercapto, alhylthio, nitro or cyano.

9. A compound according to claim 8 wherein,
R₂ is
C₂ to C₁₀ alkyl optionally substituted with one or two groups selected from hydroxy and halogen;
R and R' independently represent hydrogen or lower alkyl;
R" represents hydrogen or C₂ to C₁₀ alkyl;
R₃ is halogen, -NR₄R₅ or a moiety having the general formula: 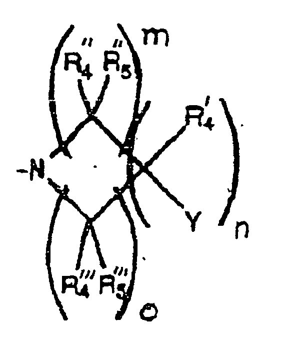
where
m, n, and o independently represent 1, 2 or 3;
Y is -NR₄R₅, hydroxy, mercapto, -OR, alkylthio ; and
R₄, R₄', R₄", R₄''', R₅, R₅" and R₅''' independently are hydrogen,
lower alkyl, optionally substituted with one, two or three groups selected from hydroxy, NRR', or
aryl, heteroaryl, or arylalkyl;
Y and R₄' together may be a single oxygen atom; or
R₄" and R₅" together may be a single oxygen atom; or
R₄"' and R₅"' together may be a single oxygen atom.

10. A compound according to claim 8 wherein
R₂ is
C₂ to C₁₀ alkyl
R and R' independently represent hydrogen, or lower alkyl;
R" represents hydrogen or C₂ to C₁₀ alkyl;
R₃ is halogen, or -NR₄R₅, where
R₄ and R₅ independetly are hydrogen, or
lower alkyl, optionally substituted with one or two groups selected from hydroxy, -NRR', or
aryl, heteroaryl, or arylalkyl.

11. A compound according to claim 8 wherein:
R₂ is C₂ to C₁₀ alkyl;
R and R' independently represent hydrogen or lower alkyl;
R" represents hydrogen or C₂ to C₁₀ alkyl;
R₃ is halogen or NR₄R₅ where
R₄ and R₅ independently are hydrogen, or
lower alkyl, optionally substituted with one or two groups selected from hydroxy, -NRR', or
heteroaryl, or arylalkyl
and
Ar is phenyl substituted with one, two or three groups selected from bromo, iodo, -OR", thienyl, pyridyl, piperonyl, or
phenyl, where the phenyl is unsubstituted or substituted with trifluoromethyl, halogen, lower alkyl, lower alkoxy, hydroxy, amino, nitro or cyano.

12. A compound according to claim 8 wherein:
R₂ is C₂ to C₁₀ alkyl ;
R and R' independently represent hydrogen or lower alkyl;
R" represents hydrogen or C₂ to C₁₀ alkyl;
R₃ is halogen, or -NR₄R₅ where
R₄ and R₅ independently are hydrogen, or
lower alkyl optionally substituted with one or two groups selected from hydroxy, -NRR', or
arylalkyl; and
Ar is phenyl substituted with one, two or three groups selected from bromo, iodo, -OR", thienyl, pyridyl, piperonyl, or-
phenyl, where the phenyl is unsubstituted or substituted with trifluoromethyl, halogen, lower alkyl, lower alkoxy, hydroxy, amino, nitro or cyano.

13. A compound according to claim 7
wherein R₁ is NHCH₂Ar;
Ar is phenyl substituted with one, two or three groups selected from halogen, -OR, thienyl, nitro, pyridyl, piperonyl, or
phenyl, where the phenyl is unsubstituted or substituted with trifluoromethyl, halogen, lower alkyl, lower alkoxy, hydroxy, amino, mercapto, alkylthio., nitro or cyano.

14. A compound according to claim 13 wherein
R₂ is
C₂ to C₁₀ alkyl optionally substituted with one or two groups selected from hydroxy, or halogen;
R and R' independently represent hydrogen, or lower alkyl;
R₃ is halogen, -NR₄R₅ or a moiety having the general formula: 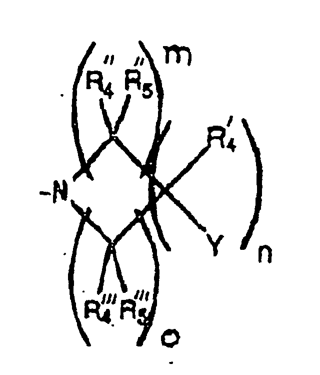
where
m, n, and o independently represent 1, 2 or 3;
Y is -NR₄R₅, hydroxy, mercapto, -OR, alkylthio ; and
R₄, R₄', R₄", R₄''', R₅, R₅" and R₅''' independently are hydrogen, or
lower alkyl, optionally substituted with one, two or three groups selected from hydroxy, -NRR', or
aryl, heteroaryl, or arylalkyl; or
Y and R₄' together may be a single oxygen atom; or
R₄" and R₅" together may be a single oxygen atom; or
R₄''' and R₅''' together may be a single oxygen atom; and
Ar is phenyl substituted with one, two or three groups selected from halogen, -OR, thienyl, nitro, pyridyl, piperonyl, or
phenyl, where the phenyl is unsubstituted or substituted with trifluoromethyl, halogen, lower alkyl, lower alkoxy, hydroxy, amino, mercapto, alkylthio, nitro or cyano.

15. A compound according to claim 13 wherein
R₂ is
C₂ to C₁₀ alkyl;
R and R' independently represent hydrogen, or lower alkyl;
R₃ is halogen, or -NR₄R₅ where
R₄ and R₅ independently are hydrogen, or
lower alkyl, optionally substituted with one or two groups selected from hydroxy, -NRR', or
aryl, heteroaryl, or arylalkyl.

16. A compound according to claim 13 wherein
R₂ is C₂ to C₁₀ alkyl;
R and R' independently represent hydrogen, or lower alkyl;
R₃ is halogen or -NR₄R₅ where
R₄ and R₅ independently are hydrogen, or
lower alkyl, optionally substituted with one or two groups selected from hydroxy, -NRR', or
heteroaryl, or arylalkyl;
Ar is phenyl substituted with one, two or three groups selected from halogen, -OR, thienyl, pyridyl, piperonyl, or
phenyl, where the phenyl is unsubstituted or substituted with trifluoromethyl, halogen, lower alkyl, lower alkoxy, hydroxy, amino, nitro or cyano.

17. A compound of claim 13 wherein
R₂ is C₂ to C₁₀ alkyl ;
R and R' independently represent hydrogen or lower alkyl;
R₃ is halogen, or -NR₄R₅, where
R₄ and R₅ independently represent hydrogen, or
lower alkyl, optionally substituted with one or two groups selected from hydroxy, -NRR', or
arylalkyl; and
Ar is phenyl substituted with one, two or three groups selected from halogen, -OR, thienyl, pyridyl, piperonyl, or
phenyl, where the phenyl is unsubstituted or substituted with trifluoromethyl, halogen, lower alkyl, lower alkoxy, hydroxy, amino, nitro or cyano.

18. A compound according to claim 7
wherein R₁ is NHR₁' and R₁' is quinolin-3-yl.

19. A compound according to claim 8 wherein Ar is 3-iodophenyl, 4-bromophenyl or 4-biphenyl.

20. A compound according to of claim 13 wherein CH₂Ar is 4-phenylbenzyl, 4-methoxybenzyl, 3-methoxybenzyl, 4-(2-thienyl)benzyl, 4-(4-methyl)phenylbenzyl, 4-(4-trifluoromethyl)phenylbenzyl, 3-(4-cyanophenylbenzyl), 4-(4-cyanophenyl)benzyl, 4-(2-pyridyl)benzyl, 3-methoxybenzyl, 2-chlorobenzyl, 3-chlorobenzyl, 4-chlorobenzyl, 2,5-difluorobenzyl, 4-and 4-nitrobenzyl.

21. A compound according to claim 11 wherein R₃ is chloro, diethanolamino, diisopropanolamino, 2-aminoethylamino, 2-aminopropylamino, 2-(methylamino)ethylamino, 1-hydroxymethyl-2-methylpropylamine or 3-amino-1,2-propandiol and R₂ is isopropyl.

22. A compound according to claim 21 wherein R₃ is chloro, diethanolamino, diisopropanolamino, 2-aminoethylamino, 2-aminopropylamino, 1-hydroxymethyl-2-methylpropylamine or 3-amino-1,2-propandiol and R₂ is isopropyl.

23. A compound according to claim 7 which is
2-{(2-hydroxyethyl)-[9-isopropyl-6-(4-methoxybenzylamino)-9-H-purine-2-yl]-amino}-ethanol; or
2-[[6-(4-bromobenzylamino)-9-isopropyl-9-H-purine-2-yl]-(2-hydroxyethyl)-amino]-ethanol].

24. A cationic salt of a compound according to claim 1 or 7.

25. An acid addition salt of a compound according to claim 1 or 7.

26. Use of therapeutically effective amount of a compound according to any one of claims 7 to 25 for the preparation of a pharmaceutical composition for inhibiting cell proliferation in a mammal.

27. The use according to claim 26 wherein the therapeutically effective amount of the compound ranges from 0.001 to 100 mg/kg weight of the mammal.

28. The use according to claim 26 or 27 wherein the composition is suitable for administration to a mammal suffering from a cell proliferation disorder selected from the group consisting of rheumatoid arthritis, lupus, type I diabetes, multiple sclerosis, cancer, restenosis, host graft disease, gout and polycystic kidney disease.

29. The use according to any one of claims 26 to 28 wherein the mammal is a human.

30. A pharmaceutical composition comprising a compound according to any one of claims 7 to 25 and one or more pharmaceutical excipients.

31. The pharmaceutical composition according to claim 30 in the form of a solution.

32. The pharmaceutical composition according to claim 30 in the form of a tablet.

33. An antifungal agent useful for treating fungal infections in humans, animals and plants comprising a compound according to any one of claims 7 to 25.

## Patentansprüche

1. Verbindung der allgemeinen Formel: 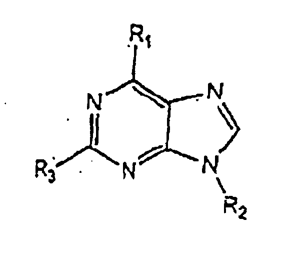 oder ein pharmazeutisch verträgliches Salz davon, worin
R₁ ein Halogenatom ist,
R₂ eine C₂- bis C₁₀-Alkylgruppe, gegebenenfalls substituiert mit einer, zwei oder drei Gruppen, ausgewählt aus Hydroxygruppe, Halogenatom und -C(O)R-Gruppe, ist,
R und R' unabhängig voneinander ein Wasserstoffatom oder
eine Niederalkylgruppe, gegebenenfalls substituiert mit einer, zwei oder drei Gruppen, ausgewählt aus Hydroxy-, -O-Alkylgruppe, Halogenatom, Amino-, Mono- oder Dialkylamino-, Mercapto-, Alkylthiol-, -C(O)NH-Alkyl-, -C(O)N-Dialkyl-, -C(O)O-Alkyl-, Cyan-, Aryloxy-, Alkenyl-, Alkinyl-, -C(O)Alkylgruppe,
oder
Aryl- oder Heteroarylgruppe, gegebenenfalls substituiert mit einer, zwei oder drei Gruppen, ausgewählt aus Niederalkyl-, Alkoxygruppe, Halogenatom, Mercapto-, Alkylthio-, Acetylen-, Amino-, Mono- oder Dialkylamino-, -C(O)NH-Alkyl-, -C(O)N-Dialkyl-, -C(O)O-Alkyl-, -C(O)Alkyl-, Hydroxy-, Aryl-, Aryloxy-, Heteroaryl-, Nitro- oder Cyangruppe, sind und
R₃ ein Halogenatom, eine -NR₄R₅-Gruppe oder eine Gruppe der allgemeinen Formel: 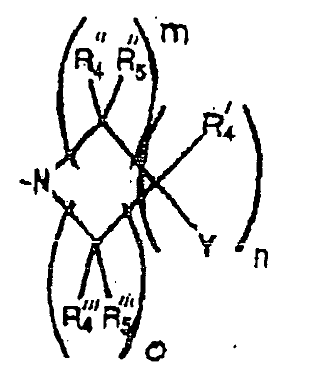
ist, worin m, n und o unabhängig voneinander den Wert 1, 2 oder 3 aufweisen,
Y eine -NR₄R₅-, Hydroxy-, Mercapto-, -OR-, Alkylthiolgruppe ist und
R₄, R₄', R₄", R₄''', R₅, R₅" und R₅''' jeweils unabhängig voneinander ein Wasserstoffatom, eine Hydroxygruppe,
Niederalkylgruppe, gegebenenfalls substituiert mit einer, zwei oder drei Gruppen, ausgewählt aus Hydroxy-, NRR'-Gruppe oder
Aryl-, Heteroaryl- oder Arylalkylgruppe sind, oder
Y und R₄' zusammen ein einzelnes Sauerstoffatom sein können oder
R₄" und R₅" zusammen ein einzelnes Sauerstoffatom sein können oder
R₄''' und R₅''' zusammen ein einzelnes Sauerstoffatom sein können.

2. Verbindung gemäß Anspruch 1, wobei R₁ ein Chloratom ist.

3. Verbindung gemäß Anspruch 2, wobei R₂ eine C₂- bis C₁₀-Alkylgruppe ist, die gegebenenfalls mit einer, zwei oder drei Hydroxygruppen substituiert ist.

4. Verbindung gemäß Anspruch 2, wobei R₃ ein Halogenatom oder eine -NR₄R₅-Gruppe ist.

5. Verbindung gemäß Anspruch 4, wobei R₃ ein Chloratom ist.

6. Verbindung gemäß Anspruch 4, wobei R₃ eine -NR₄R₅-Gruppe ist.

7. Verbindung der allgemeinen Formel 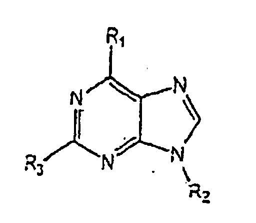 oder ein pharmazeutisch verträgliches Salz davon, worin
R₁ eine -NHR₁'-Gruppe ist,
R₁' eine Chinolin-3-yl- oder Chinolin-6-ylgruppe ist oder
R₁' eine Benzylgruppe ist, wobei der Ringanteil mit einer, zwei oder drei Gruppen substituiert ist, ausgewählt aus Halogenatom, -OR-, Thienyl-, Nitro-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Mercapto-, Alkylthio-, Nitro- oder Cyangruppe substituiert ist, oder
R₁' eine Phenylgruppe, substituiert mit einer, zwei oder drei Gruppen, ausgewählt aus Brom-, Iodatom, -OR"-, Thienyl-, Pyridyl-, Piperonylgruppe oder Phenylgruppe, wobei die Phenylgruppe nicht substituiert oder mit Trifluormethylgruppe, Halogenatom, Niederalkyl-, Niederalkoxy-, Hydroxy-, Amino-, Mercapto-, Alkylthio-, Nitro- oder Cyangruppe substituiert ist, ist,
R₂ eine C₂- bis C₁₀-Alkylgruppe, gegebenenfalls substituiert mit einer, zwei oder drei Gruppen, ausgewählt aus Hydroxygruppe, Halogenatom und -C(O)R-Gruppe, ist,
R und R' unabhängig voneinander ein Wasserstoffatom oder
eine Niederalkylgruppe, gegebenenfalls substituiert mit einer, zwei oder drei Gruppen, ausgewählt aus Hydroxy-, -O-Alkylgruppe, Halogenatom, Amino-, Mono- oder Dialkylamino-, Mercapto-, Alkylthiol-, -C(O)NH-Alkyl-, -C(O)N-Dialkyl-, -C(O)O-Alkyl-, Cyan-, Aryloxy-, Alkenyl-, Alkinyl-, -C(O)Alkylgruppe,
oder
Aryl- oder Heteroarylgruppe, gegebenenfalls substituiert mit einer, zwei oder drei Gruppen, ausgewählt aus Niederalkyl-, Alkoxygruppe, Halogenatom, Mercapto-, Alkylthiol-, Acetylen-, Amino-, Mono- oder Dialkylamino-, -C(O)NH-Alkyl-, -C(O)N-Dialkyl-, -C(O)O-Alkyl-, -C(O)Alkyl-, Hydroxy-, Aryl-, Aryloxy-, Heteroaryl-, Nitro- oder Cyangruppe, sind,
R" ein Wasserstoffatom oder
eine C₂- bis C₁₀-Alkylgruppe, gegebenenfalls substituiert mit einer, zwei oder drei Gruppen, ausgewählt aus Hydroxy-, -O-Alkylgruppe, Halogenatom, Amino-, Mono- oder Dialkylamino-, Mercapto-, Alkylthiol-, -C(O)NH-Alkyl-, -C(O)N-Dialkyl-, -C(O)O-Alkyl-, Cyan-, Aryloxy-, Alkenyl-, Alkinyl-, -C(O)Alkylgruppe oder Aryl- oder Heteroarylgruppe, gegebenenfalls substituiert mit einer, zwei oder drei Gruppen, ausgewählt aus Niederalkyl-, Alkoxygruppe, Halogenatom, Mercapto-, Alkylthiol-, Acetylen-, Amino-, Mono- oder Dialkylamino-, -C(O)NH-Alkyl-, -C(O)N-Dialkyl-, -C(O)O-Alkyl-, -C(O)Alkyl-, Hydroxy-, Aryl-, Aryloxy-, Heteroaryl-, Nitro- oder Cyangruppe, ist,
R₃ ein Halogenatom, eine -NR₄R₅-Gruppe oder eine Gruppe der allgemeinen Formel: 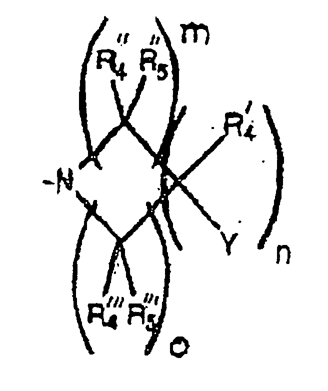
ist, worin m, n und o unabhängig voneinander den Wert 1, 2 oder 3 aufweisen,
Y eine -NR₄R₅-, Hydroxy-, Mercapto-, -OR-, Alkylthiogruppe ist und
R₄, R₄', R₄", R₄''', R₅, R₅" und R₅''' unabhängig voneinander ein Wasserstoffatom, eine Hydroxygruppe,
Niederalkylgruppe, gegebenenfalls substituiert mit einer, zwei oder drei Gruppen, ausgewählt aus Hydroxy-, -NRR'-Gruppe oder
Aryl-, Heteroaryl- oder Arylalkylgruppe sind, oder
Y und R₄' zusammen ein einzelnes Sauerstoffatom sein können oder
R₄" und R₅" zusammen ein einzelnes Sauerstoffatom sein können oder
R₄''' und R₅''' zusammen ein einzelnes Sauerstoffatom sein können.

8. Verbindung gemäß Anspruch 7,
wobei R₁ eine NHAr-Gruppe ist und
Ar eine Phenylgruppe, substituiert mit einer, zwei oder drei Gruppen, ausgewählt aus Brom-, Iodatom, -OR"-, Thienyl-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Mercapto-, Alkylthio-, Nitro- oder Cyangruppe substituiert ist, ist.

9. Verbindung gemäß Anspruch 8, wobei
R₂ eine C₂- bis C₁₀-Alkylgruppe ist, gegebenenfalls substituiert mit einer oder zwei Gruppen, ausgewählt aus Hydroxygruppe und Halogenatom,
R und R' unabhängig voneinander ein Wasserstoffatom oder eine Niederalkylgruppe sind,
R" ein Wasserstoffatom oder eine C₂- bis C₁₀-Alkylgruppe ist,
R₃ ein Halogenatom, eine -NR₄R₅-Gruppe oder eine Gruppe der allgemeinen Formel: 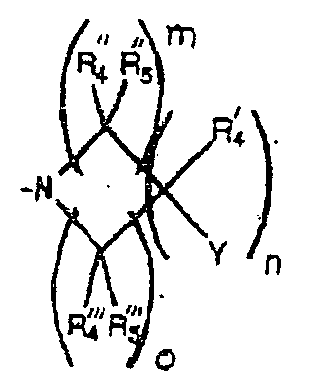
ist, worin m, n und o unabhängig voneinander den Wert von 1, 2 oder 3 aufweisen,
Y eine -NR₄R₅-, Hydroxy-, Mercapto-, -OR-, Alkylthiogruppe ist und
R₄, R₄', R₄", R₄''', R₅, R₅" und R₅''' unabhängig voneinander ein Wasserstoffatom, eine Niederalkylgruppe, gegebenenfalls substituiert mit einer, zwei oder drei Gruppen, ausgewählt aus Hydroxy-, NRR'-Gruppe oder Aryl-, Heteroaryl- oder Arylalkylgruppe sind,
Y und R₄' zusammen ein einzelnes Sauerstoffatom sein können oder
R₄" und R₅" zusammen ein einzelnes Sauerstoffatom sein können oder
R₄''' und R₅''' zusammen ein einzelnes Sauerstoffatom sein können.

10. Verbindung gemäß Anspruch 8, wobei
R₂ eine C₂- bis C₁₀-Alkylgruppe ist,
R und R' unabhängig voneinander ein Wasserstoffatom oder eine Niederalkylgruppe sind,
R" ein Wasserstoffatom oder eine C₂- bis C₁₀-Alkylgruppe ist,
R₃ ein Halogenatom oder eine -NR₄R₅-Gruppe ist, worin
R₄ und R₅ unabhängig voneinander ein Wasserstoffatom oder
eine Niederalkylgruppe, gegebenenfalls substituiert mit einer oder zwei Gruppen, ausgewählt aus Hydroxy-, -NRR'-Gruppe oder Aryl-, Heteroaryl- oder Arylalkylgruppe sind.

11. Verbindung gemäß Anspruch 8, wobei:
R₂ eine C₂- bis C₁₀-Alkylgruppe ist,
R und R' unabhängig voneinander ein Wasserstoffatom oder eine Niederalkylgruppe sind,
R" ein Wasserstoffatom oder eine C₂- bis C₁₀-Alkylgruppe ist,
R₃ ein Halogenatom oder eine NR₄R₅-Gruppe ist, worin
R₄ und R₅ unabhängig voneinander ein Wasserstoffatom oder
eine Niederalkylgruppe, gegebenenfalls substituiert mit einer oder zwei Gruppen, ausgewählt aus Hydroxy-, -NRR'-Gruppe oder
Heteroaryl- oder Arylalkylgruppe sind, und
Ar eine Phenylgruppe, substituiert mit einer, zwei oder drei Gruppen, ausgewählt aus Brom-, Iodatom, -OR"-, Thienyl-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Nitro- oder Cyangruppe substituiert ist, ist.

12. Verbindung gemäß Anspruch 8, wobei:
R₂ eine C₂- bis C₁₀-Alkylgruppe ist,
R und R' unabhängig voneinander ein Wasserstoffatom oder eine Niederalkylgruppe sind,
R" ein Wasserstoffatom oder eine C₂- bis C₁₀-Alkylgruppe ist,
R₃ ein Halogenatom oder eine -NR₄R₅-Gruppe ist, worin
R₄ und R₅ unabhängig voneinander ein Wasserstoffatom oder
eine Niederalkylgruppe, gegebenenfalls substituiert mit einer oder zwei Gruppen, ausgewählt aus Hydroxy-, -NRR'-Gruppe oder
Arylalkylgruppe sind, und
Ar eine Phenylgruppe, substituiert mit einer, zwei oder drei Gruppen, ausgewählt aus Brom-, Iodatom, -OR"-, Thienyl-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Nitro- oder Cyangruppe substituiert ist, ist.

13. Verbindung gemäß Anspruch 7,
wobei R₁ eine NHCH₂Ar-Gruppe ist,
Ar eine Phenylgruppe, substituiert mit einer, zwei oder drei Gruppen, ausgewählt aus Halogenatom, -OR-, Thienyl-, Nitro-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Mercapto-, Alkylthio-, Nitro- oder Cyangruppe substituiert ist, ist.

14. Verbindung gemäß Anspruch 13, wobei
R₂ eine C₂- bis C₁₀-Alkylgruppe ist, gegebenenfalls substituiert mit einer oder zwei Gruppen, ausgewählt aus Hydroxygruppe oder Halogenatom,
R und R' unabhängig voneinander ein Wasserstoffatom oder eine Niederalkylgruppe sind,
R₃ ein Halogenatom, eine -NR₄R₅-Gruppe oder eine Gruppe der allgemeinen Formel: 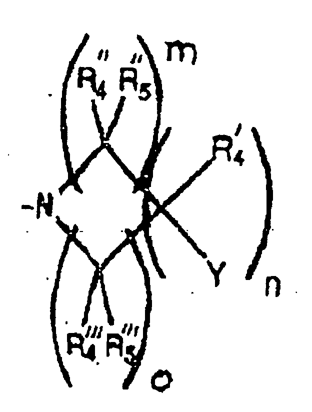
ist, worin m, n und o unabhängig voneinander den Wert 1, 2 oder 3 aufweisen,
Y eine -NR₄R₅-, Hydroxy-, Mercapto-, -OR-, Alkylthiogruppe ist und
R₄, R₄', R₄", R₄''', R₅, R₅" und R₅''' unabhängig voneinander ein Wasserstoffatom oder
eine Niederalkylgruppe, gegebenenfalls substituiert mit einer, zwei oder drei Gruppen, ausgewählt aus Hydroxy-, -NRR'-Gruppe oder
Aryl-, Heteroaryl- oder Arylalkylgruppe sind, oder
Y und R₄' zusammen ein einzelnes Sauerstoffatom sein können oder
R₄" und R₅" zusammen ein einzelnes Sauerstoffatom sein können oder
R₄''' und R₅''' zusammen ein einzelnes Sauerstoffatom sein können und
Ar eine Phenylgruppe, substituiert mit einer, zwei oder drei Gruppen, ausgewählt aus Halogenatom, -OR-, Thienyl-, Nitro-, Pyridyl-, Piperonylgruppe oder Phenylgruppe, wobei die Phenylgruppe nicht substituiert oder mit Trifluormethylgruppe, Halogenatom, Niederalkyl-, Niederalkoxy-, Hydroxy-, Amino-, Mercapto-, Alkylthio-, Nitro- oder Cyangruppe substituiert ist, ist.

15. Verbindung gemäß Anspruch 13, wobei
R₂ eine C₂- bis C₁₀-Alkylgruppe ist,
R und R' unabhängig voneinander ein Wasserstoffatom oder eine Niederalkylgruppe sind,
R₃ ein Halogenatom oder eine -NR₄R₅-Gruppe ist, worin
R₄ und R₅ unabhängig voneinander ein Wasserstoffatom oder eine Niederalkylgruppe, gegebenenfalls substituiert mit einer oder zwei Gruppen, ausgewählt aus Hydroxy-, -NRR'-Gruppe oder Aryl-, Heteroaryl- oder Arylalkylgruppe sind.

16. Verbindung gemäß Anspruch 13, wobei
R₂ eine C₂- bis C₁₀-Alkylgruppe ist,
R und R' unabhängig voneinander ein Wasserstoffatom oder eine Niederalkylgruppe sind,
R₃ ein Halogenatom oder eine -NR₄R₅-Gruppe ist, worin
R₄ und R₅ unabhängig voneinander ein Wasserstoffatom oder eine Niederalkylgruppe, gegebenenfalls substituiert mit einer oder zwei Gruppen, ausgewählt aus Hydroxy-, -NRR'-Gruppe oder
Heteroaryl- oder Arylalkylgruppe sind, und
Ar eine Phenylgruppe, substituiert mit einer, zwei oder drei Gruppen, ausgewählt aus Halogenatom, -OR-, Thienyl-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Nitro- oder Cyangruppe substituiert ist, ist.

17. Verbindung gemäß Anspruch 13, wobei
R₂ eine C₂- bis C₁₀-Alkylgruppe ist,
R und R' unabhängig voneinander ein Wasserstoffatom oder eine Niederalkylgruppe sind,
R₃ ein Halogenatom oder eine -NR₄R₅-Gruppe ist, worin
R₄ und R₅ unabhängig voneinander ein Wasserstoffatom oder eine Niederalkylgruppe, gegebenenfalls substituiert mit einer oder zwei Gruppen, ausgewählt aus Hydroxy-, -NRR'-Gruppe oder
Arylalkylgruppe sind, und
Ar eine Phenylgruppe, substituiert mit einer, zwei oder drei Gruppen, ausgewählt aus Halogenatom, -OR-, Thienyl-, Pyridyl-, Piperonylgruppe oder
Phenylgruppe, wobei die Phenylgruppe nicht substituiert oder mit Trifluormethylgruppe, Halogenatom, Niederalkyl-, Niederalkoxy-, Hydroxy-, Amino-, Nitro- oder Cyangruppe substituiert ist, ist.

18. Verbindung gemäß Anspruch 7,
wobei R₁ eine NHR₁'-Gruppe ist und R₁' eine Chinolin-3-ylgruppe ist.

19. Verbindung gemäß Anspruch 8, wobei Ar eine 3-Iodphenyl-, 4-Bromphenyl- oder 4-Biphenylgruppe ist.

20. Verbindung gemäß Anspruch 13, wobei CH₂Ar eine 4-Phenylbenzyl-, 4-Methoxybenzyl-, 3-Methoxybenzyl-, 4-(2-Thienyl)benzyl-, 4-(4-Methyl)phenylbenzyl-, 4-(4-Trifluormethyl)phenylbenzyl-, 3-(4-Cyanphenylbenzyl)-, 4-(4-Cyanphenyl)benzyl-, 4-(2-Pyridyl)benzyl-, 3-Methoxybenzyl-, 2-Chlorbenzyl-, 3-Chlorbenzyl-, 4-Chlorbenzyl-, 2,5-Difluorbenzyl- und 4-Nitrobenzylgruppe ist.

21. Verbindung gemäß Anspruch 11, wobei R₃ ein Chloratom, eine Diethanolamino-, Diisopropanolamino-, 2-Aminoethylamino-, 2-Aminopropylamino-, 2-(Methylamino)ethylamino-, 1-Hydroxymethyl-2-methylpropylamin- oder 3-Amino-1,2-propandiolgruppe ist und R₂ eine Isopropylgruppe ist.

22. Verbindung gemäß Anspruch 21, wobei R₃ ein Chloratom, eine Diethanolamino-, Diisopropanolamino-, 2-Aminoethylamino-, 2-Aminopropylamino-, 1-Hydroxymethyl-2-methylpropylamin- oder 3-Amino-1,2-propandiolgruppe ist und R₂ eine Isopropylgruppe ist.

23. Verbindung gemäß Anspruch 7, die
2-{(2-Hydroxyethyl)-[9-isopropyl-6-(4-methoxybenzylamino)-9-H-purin-2-yl]amino}ethanol oder
2-[[6-(4-Brombenzylamino)-9-isopropyl-9-H-purin-2-yl]-(2-hydroxyethyl)amino]-ethanol ist.

24. Kationisches Salz einer Verbindung gemäß Anspruch 1 oder 7.

25. Säure-Additionssalz einer Verbindung gemäß Anspruch 1 oder 7.

26. Verwendung einer therapeutisch wirksamen Menge einer Verbindung gemäß einem der Ansprüche 7 bis 25 zur Herstellung einer pharmazeutischen Zusammensetzung zum Hemmen der Zellproliferation in einem Säuger.

27. Verwendung gemäß Anspruch 26, wobei die therapeutisch wirksame Menge der Verbindung 0,001 bis 100 mg/kg Gewicht des Säugers beträgt.

28. Verwendung gemäß Anspruch 26 oder 27, wobei die Zusammensetzung zum Verabreichen an einen Säuger geeignet ist, der an einer Zellproliferationserkrankung leidet, ausgewählt aus der Gruppe bestehend aus rheumatoider Arthritis, Lupus, Typ I-Diabetes, multipler Sklerose, Krebs, Restenose, Wirt-Transplantat-Erkrankung, Gicht und polyzystischer Nierenerkrankung.

29. Verwendung gemäß einem der Ansprüche 26 bis 28, wobei der Säuger ein Mensch ist.

30. Pharmazeutische Zusammensetzung, die eine Verbindung gemäß einem der Ansprüche 7 bis 25 und ein oder mehrere pharmazeutische Exzipienzien umfasst.

31. Pharmazeutische Zusammensetzung gemäß Anspruch 30 in der Form einer Lösung.

32. Pharmazeutische Zusammensetzung gemäß Anspruch 30 in der Form einer Tablette.

33. Antipilzmittel, das zum Behandeln von Pilzinfektionen in Menschen, Tieren und Pflanzen geeignet ist und das eine Verbindung gemäß einem der Ansprüche 7 bis 25 umfasst.

## Revendications

1. Composé de formule générale : 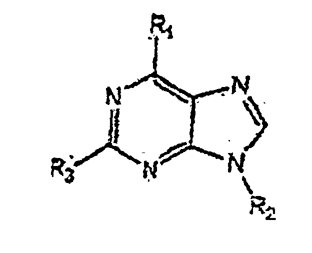 ou un de ses sels pharmaceutiquement acceptables, formule
dans laquelle
R₁ représente un atome d'halogène ;
R₂ représente
un groupe alkyle en C₂ à C₁₀ facultativement substitué avec un, deux ou trois groupes choisis entre des groupes hydroxy, halogéno et -C(O)R ;
R et R' représentent indépendamment un atome d'hydrogène, ou
un groupe alkyle inférieur facultativement substitué avec un, deux ou trois groupes choisis entre des groupes hydroxy, -O-alkyle, halogéno, amino, mono- ou dialkylamino, mercapto, alkylthiol, -C(O)NH-alkyle, -C(O)N-dialkyle, -C(O)O-alkyle, cyano, aryl-oxy, alcényle, alcynyle, -C(O)alkyle, ou
un groupe aryle ou hétéroaryle facultativement substitué avec un, deux ou trois groupes choisis entre des groupes alkyle inférieurs, alkoxy, halogéno, mercapto, alkylthio, acétylène, amino, mono- ou dialkylamino, -C(O)NH-alkyle, -C(O)N-dialkyle, -C(O)O-alkyle, -C(O)alkyle, hydroxy, aryle, aryloxy, hétéroaryle, nitro ou cyano ; et
R₃ représente un atome d'halogène, un groupe -NR₄R₅ ou un groupement de formule générale : 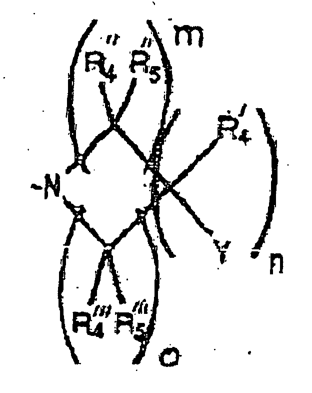 dans laquelle m, n et o sont égaux indépendamment à 1, 2 ou 3,
Y représente un groupe -NR₄R₅, hydroxy, mercapto, -OR, alkylthiol ; et
R₄, R₄', R₄", R₄''', R₅, R₅" et R₅''' représentent chacun indépendamment un atome d'hydrogène, un groupe hydroxy,
alkyle inférieur, facultativement substitué avec un, deux ou trois groupes choisis entre des groupes hydroxy, NRR', ou
aryle, hétéroaryle ou arylalkyle ; ou
Y et R₄', conjointement, peuvent représenter un atome d'oxygène unique ; ou
R₄" et R₅", conjointement, peuvent représenter un atome d'oxygène unique ; ou
R₄''' et R₅''', conjointement, peuvent représenter un atome d'oxygène unique.

2. Composé suivant la revendication 1, dans lequel R₁ représente un groupe chloro.

3. Composé suivant la revendication 2, dans lequel R₂ représente un groupe alkyle en C₂ à C₁₀ facultativement substitué avec un, deux ou trois groupes hydroxy.

4. Composé suivant la revendication 2, dans lequel R₃ représente un atome d'halogène ou un groupe -NR₄R₅.

5. Composé suivant la revendication 4, dans lequel R₃ représente un groupe chloro.

6. Composé suivant la revendication 4, dans lequel R₃ représente un groupe -NR₄R₅.

7. Composé de formule générale 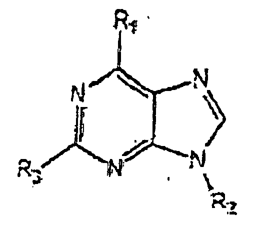 ou un de ses sels pharmaceutiquement acceptables, formule
dans laquelle
R₁ représente un groupe -NHR₁' ;
R₁' représente un groupe quinoléine-3-yle ou quinoléine-6-yle ; ou
R₁' représente un groupe benzyle dans lequel la portion constituée du noyau est substituée avec un, deux ou trois groupes choisis entre des groupes halogéno, -OR, thiényle, nitro, pyridyle, pipéronyle, ou
phényle, le groupe phényle étant non substitué ou substitué avec des substituants trifluorométhyle, halogéno, alkyle inférieur, alkoxy inférieur, hydroxy, amino, mercapto, alkylthio, nitro ou cyano ; ou
R₁' représente un groupe phényle substitué avec un, deux ou trois groupes choisis entre des groupes bromo, iodo, -OR", thiényle, pyridyle, pipéronyle, ou
phényle, le groupe phényle étant non substitué ou substitué avec des groupes trifluorométhyle, halogéno, alkyle inférieur, alkoxy inférieur, hydroxy, amino, mercapto, alkylthio, nitro ou cyano ;
R₂ représente un groupe alkyle en C₂ à C₁₀ facultativement substitué avec un, deux ou trois groupes choisis entre des groupes hydroxy, halogène et -C(O)R ;
R et R' représentent indépendamment un atome d'hydrogène, ou
un groupe alkyle inférieur, facultativement substitué avec un, deux ou trois groupes choisis entre des groupes hydroxy, -O-alkyle, halogéno, amino, mono- ou dialkylamino, mercapto, alkylthiol, -C(O)NH-alkyle, -C(O)N-dialkyle, -C(O)O-alkyle, cyano, aryloxy, alcényle, alcynyle, -C(O)alkyle, ou
un groupe aryle ou hétéroaryle facultativement substitué avec un, deux ou trois groupes choisis entre des groupes alkyle inférieur, alkoxy, halogéno, mercapto, alkylthiol, acétylène, amino, mono- ou dialkylamino, -C(O)NH-alkyle, -C(O)N-dialkyle, -C(O)O-alkyle, -C(O)alkyle, hydroxy, aryle, aryloxy, hétéroaryle, nitro ou cyano ;
R" représente un atome d'hydrogène, ou
un groupe alkyle en C₂ à C₁₀, facultativement substitué avec un, deux ou trois groupes choisis entre des groupes hydroxy, -O-alkyle, halogéno, amino, mono- ou dialkylamino, mercapto, alkylthiol, -C(O)NH-alkyle, -C(O)N-dialkyle, -C(O)O-alkyle, cyano, aryloxy, alcényle, alcynyle, -C(O)alkyle, ou
un groupe aryle ou hétéroaryle facultativement substitué avec un, deux ou trois groupes choisis entre des groupes alkyle inférieur, alkoxy, halogéno, mercapto, alkylthiol, acétylène, amino, mono- ou dialkylamino, -C(O)NH-alkyle, -C(O)N-dialkyle, -C(O)O-alkyle, -C(O)alkyle, hydroxy, aryle, aryloxy, hétéroaryle, nitro ou cyano ;
R₃ représente un atome d'halogène, un groupe -NR₄R₅ ou un groupement de formule générale : 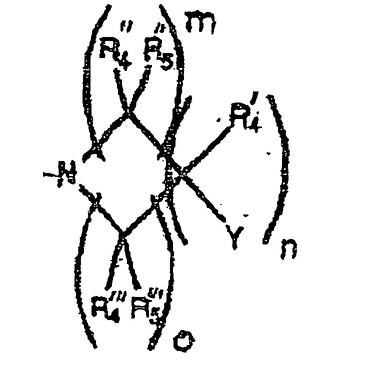 dans laquelle m, n et o sont égaux indépendamment à 1, 2 ou 3 ;
Y représente un groupe -NR₄R₅, hydroxy, mercapto, -OR, alkylthio ; et
R₄, R₄', R₄", R₄''', R₅, R₅" et R₅''' représentent, indépendamment, un atome d'hydrogène, un groupe hydroxy, un groupe alkyle inférieur facultativement substitué avec un, deux ou trois groupes choisis entre des groupes hydroxy, -NRR', ou
aryle, hétéroaryle ou arylalkyle ; ou
Y et R₄', conjointement, peuvent représenter un atome d'oxygène unique ; ou
R₄" et R₅", conjointement, peuvent représenter un atome d'oxygène unique ; ou
R₄''' et R₅''', conjointement, peuvent représenter un atome d'oxygène unique.

8. Composé suivant la revendication 7,
dans lequel R₁ représente un groupe NHAr, et
Ar représente un groupe phényle substitué avec un, deux ou trois groupes choisis entre des groupes bromo, iodo, -OR", thiényle, pyridyle, pipéronyle, ou
phényle, le groupe phényle étant non substitué ou substitué avec des substituants trifluorométhyle, halogéno, alkyle inférieur, alkoxy inférieur, hydroxy, amino, mercapto, alkylthio, nitro ou cyano.

9. Composé suivant la revendication 8, dans lequel
R₂ représente
un groupe alkyle en C₂ à C₁₀ facultativement substitué avec un ou deux groupes choisis entre des groupes hydroxy et halogéno ;
R et R' représentent indépendamment un atome d'hydrogène ou un groupe alkyle inférieur ;
R" représente un atome d'hydrogène ou un groupe alkyle en C₂ à C₁₀ ;
R₃ représente un atome d'halogène, un groupe -NR₄R₅ ou un groupement de formule générale : 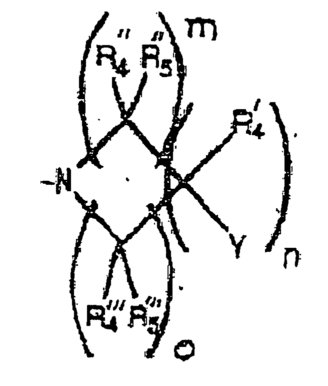 dans laquelle m, n et o sont égaux indépendamment à 1, 2 ou 3 ;
Y représente un groupe -NR₄R₅, hydroxy, mercapto, -OR, alkylthio ; et
R₄, R₄', R₄", R₄''', R₅, R₅" et R₅''' représentent, indépendamment, un atome d'hydrogène, un groupe alkyle inférieur, facultativement substitué avec un, deux ou trois groupes choisis entre des groupes hydroxy, -NRR', ou aryle, hétéroaryle ou arylalkyle ;
Y et R₄', conjointement, peuvent représenter un atome d'oxygène unique ; ou
R₄" et R₅", conjointement, peuvent représenter un atome d'oxygène unique ; ou
R₄''' et R₅''', conjointement, peuvent représenter un atome d'oxygène unique.

10. Composé suivant la revendication 8, dans lequel
R₂ représente
un groupe alkyle en C₂ à C₁₀;
R et R' représentent indépendamment un atome d'hydrogène ou un groupe alkyle inférieur ;
R" représente un atome d'hydrogène ou un groupe alkyle en C₂ à C₁₀ ;
R₃ représente un atome d'halogène ou un groupe -NR₄R₅, dans lequel
R₄ et R₅ représentent indépendamment un atome d'hydrogène ou
un groupe alkyle inférieur, facultativement substitué avec un ou deux groupes choisis entre des groupes hydroxy, -NRR', ou
aryle, hétéroaryle ou arylalkyle.

11. Composé suivant la revendication 8, dans lequel :
R₂ représente un groupe alkyle en C₂ à C₁₀ ;
R et R' représentent indépendamment un atome d'hydrogène ou un groupe alkyle inférieur ;
R" représente un atome d'hydrogène ou un groupe alkyle en C₂ à C₁₀ ;
R₃ représente un atome d'halogène ou un groupe NR₄R₅ dans lequel
R₄ et R₅ représentent indépendamment un atome d'hydrogène ou
un groupe alkyle inférieur, facultativement substitué avec un ou deux groupes choisis entre des groupes hydroxy, -NRR', ou
un groupe hétéroaryle ou arylalkyle
et
Ar représente un groupe phényle substitué avec un, deux ou trois groupes choisis entre des groupes bromo, iodo, -OR", thiényle, pyridyle, pipéronyle, ou
un groupe phényle, le groupe phényle étant non substitué ou substitué avec des substituants trifluorométhyle, halogéno, alkyle inférieur, alkoxy inférieur, hydroxy, amino, nitro ou cyano.

12. Composé suivant la revendication 8, dans lequel :
R₂ représente un groupe alkyle en C₂ à C₁₀ ;
R et R' représentent indépendamment un atome d'hydrogène ou un groupe alkyle inférieur ;
R" représente un atome d'hydrogène ou un groupe alkyle en C₂ à C₁₀ ;
R₃ représente un atome d'halogène ou un groupe -NR₄R₅ dans lequel
R₄ et R₅ représentent indépendamment un atome d'hydrogène, ou
un groupe alkyle inférieur, facultativement substitué avec un ou deux groupes choisis entre des groupes hydroxy, -NRR', ou
un groupe arylalkyle ;
Ar représente un groupe phényle substitué avec un, deux ou trois groupes choisis entre des groupes bromo, iodo, -OR", thiényle, pyridyle, pipéronyle, ou
un groupe phényle, le groupe phényle étant non substitué ou substitué avec des substituants trifluorométhyle, halogéno, alkyle inférieur, alkoxy inférieur, hydroxy, amino, nitro ou cyano.

13. Composé suivant la revendication 7,
dans lequel R₁ représente un groupe NHCH₂Ar ;
Ar représente un groupe phényle substitué avec un, deux ou trois groupes choisis entre des groupes halogène, -OR, thiényle, nitro, pyridyle, pipéronyle, ou
un groupe phényle, le groupe phényle étant non substitué ou substitué avec des substituants trifluorométhyle, halogène, alkyle inférieur, alkoxy inférieur, hydroxy, amino, mercapto, alkylthio, nitro ou cyano.

14. Composé suivant la revendication 13, dans lequel
R₂ représente
un groupe alkyle en C₂ à C₁₀ facultativement substitué avec un ou deux groupes choisis entre des groupes hydroxy et halogéno ;
R et R^{l} représentent indépendamment un atome d'hydrogène ou un groupe alkyle inférieur ;
R₃ représente un atome d'halogène, un groupe -NR₄R₅ ou un groupement de formule générale : 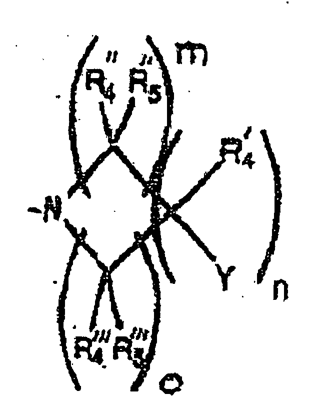 dans laquelle m, n et o sont égaux indépendamment à 1, 2 ou 3 ;
Y représente un groupe -NR₄R₅, hydroxy, mercapto, -OR, alkylthio ; et
R₄, R₄', R₄", R₄''', R₅, R₅" et R₅''' représentent, indépendamment un atome d'hydrogène ou
un groupe alkyle inférieur, facultativement substitué avec un, deux ou trois groupes choisis entre des groupes hydroxy, -NRR', ou
un groupe aryle, hétéroaryle ou arylalkyle ; ou
Y et R₄', conjointement, peuvent représenter un atome d'oxygène unique ; ou
R₄" et R₅", conjointement, peuvent représenter un atome d'oxygène unique ; ou
R₄''' et R₅''', conjointement, peuvent représenter un atome d'oxygène unique ; et
Ar représente un groupe phényle substitué avec un, deux ou trois groupes choisis entre des groupes halogéno, -OR, thiényle, nitro, pyridyle, pipéronyle, ou
un groupe phényle, le groupe phényle étant non substitué ou substitué avec des substituants trifluorométhyle, halogéno, alkyle inférieur, alkoxy inférieur, hydroxy, amino, mercapto, alkylthio, nitro ou cyano.

15. Composé suivant la revendication 13, dans lequel R₂ représente un groupe alkyle en C₂ à C₁₀ ;
R et R' représentent indépendamment un atome d'hydrogène ou un groupe alkyle inférieur ;
R₃ représente un atome d'halogène ou un groupe -NR₄R₅ dans lequel
R₄ et R₅ représentent indépendamment un atome d'hydrogène ou
un groupe alkyle inférieur, facultativement substitué avec un ou deux groupes choisis entre des groupes hydroxy, -NRR', ou
un groupe aryle, hétéroaryle ou arylalkyle.

16. Composé suivant la revendication 13, dans lequel
R₂ représente un groupe alkyle en C₂ à C₁₀ ;
R et R' représentent indépendamment un atome d'hydrogène ou un groupe alkyle inférieur ;
R₃ représente un atome d'halogène ou un groupe -NR₄R₅ dans lequel
R₄ et R₅ représentent indépendamment un atome d'hydrogène ou
un groupe alkyle inférieur, facultativement substitué avec un ou deux groupes choisis entre des groupes hydroxy, -NRR', ou
un groupe hétéroaryle ou arylalkyle, et
Ar représente un groupe phényle substitué avec un, deux ou trois groupes choisis entre des groupes halogène, -OR, thiényle, pyridyle, pipéronyle, ou
un groupe phényle, le groupe phényle étant non substitué ou substitué avec des substituants trifluorométhyle, halogéno, alkyle inférieur, alkoxy inférieur, hydroxy, amino, nitro ou cyano.

17. Composé suivant la revendication 13, dans lequel
R₂ représente un groupe alkyle en C₂ à C₁₀ ;
R et R' représentent indépendamment un atome d'hydrogène ou groupe alkyle inférieur ;
R₃ représente un atome d'halogène ou un groupe -NR₄R₅, dans lequel
R₄ et R₅ représentent indépendamment un atome d'hydrogène ou
un groupe alkyle inférieur, facultativement substitué avec un ou deux groupes choisis entre des groupes hydroxy, -NRR', ou
un groupe arylalkyle ; et
Ar représente un groupe phényle substitué avec un, deux ou trois groupes choisis entre des groupes halogéno, -OR, thiényle, pyridyle, pipéronyle, ou
un groupe phényle, le groupe phényle étant non substitué ou substitué avec des substituants trifluorométhyle, halogéno, alkyle inférieur, alkoxy inférieur, hydroxy, amino, nitro ou cyano.

18. Composé suivant la revendication 7,
dans lequel R₁ représente un groupe NHR₁' et R₁' représente un groupe quinoléine-3-yle.

19. Composé suivant la revendication 8, dans lequel Ar représente un groupe 3-iodophényle, 4-bromophényle ou 4-biphényle.

20. Composé suivant la revendication 13, dans lequel CH₂Ar représente un groupe 4-phénylbenzyle, 4-méthoxybenzyle, 3-méthoxybenzyle, 4-(2-thiényl)benzyle, 4-(4-méthyl)-phénylbenzyle, 4-(4-trifluorométhyl)phénylbenzyle, 3-(4-cyanophénylbenzyl), 4-(4-cyanophényl)benzyle, 4-(2-pyridyl)benzyle, 3-méthoxybenzyle, 2-chlorobenzyle, 3-chlorobenzyle, 4-chlorobenzyle, 2,5-difluorobenzyle et 4-nitrobenzyle.

21. Composé suivant la revendication 11, dans lequel R₃ représente un groupe chloro, diéthanolamino, diisopropanolamino, 2-aminoéthylamino, 2-aminopropylamino, 2- (méthylamino) éthylamino, 1-hydroxyméthyl-2-méthylpropylamine ou 3-amino-1,2-propanediol, et R₂ représente un groupe isopropyle.

22. Composé suivant la revendication 21, dans lequel R₃ représente un groupe chloro, diéthanolamino, diisopropanolamino, 2-aminoéthylamino, 2-aminopropylamino, 1-hydroxyméthyl-2-méthylpropylamine ou 3-amino-1,2-propanediol, et R₂ représente un groupe isopropyle.

23. Composé suivant la revendication 7, qui est
le 2-{(2-hydroxyéthyl)-[9-isopropyl-6-(4-méthoxybenzylamino)-9H-purine-2-yl]-amino}-éthanol ; ou
le 2-[[6-(4-bromobenzylamino)-9-isopropyl-9H-purine-2-yl]-(2-hydroxyéthyl)-amino]-éthanol.

24. Sel cationique d'un composé suivant la revendication 1 ou 7.

25. Sel d'addition d'acide d'un composé suivant la revendication 1 ou 7.

26. Utilisation d'une quantité thérapeutiquement efficace d'un composé suivant l'une quelconque des revendication 7 à 25 pour la préparation d'une composition pharmaceutique destinée à inhiber la prolifération cellulaire chez un mammifère.

27. Utilisation suivant la revendication 26, dans laquelle la quantité thérapeutiquement efficace du composé est comprise dans l'intervalle de 0,001 à 100 mg/kg de poids du mammifère.

28. Utilisation suivant la revendication 26 ou 27, dans laquelle la composition est apte à l'administration à un mammifère souffrant d'un trouble de la prolifération cellulaire choisi dans le groupe consistant en la polyarthrite rhumatoïde, le lupus, le diabète de type I, la sclérose en plaque, le cancer, une resténose, la réaction du greffon contre l'hôte, la goutte et la maladie rénale polykystique.

29. Utilisation suivant l'une quelconque des revendications 26 à 28, dans laquelle le mammifère est un être humain.

30. Composition pharmaceutique comprenant un composé suivant l'une quelconque des revendications 7 à 25 et un ou plusieurs excipients pharmaceutiques.

31. Composition pharmaceutique suivant la revendication 30, sous forme d'une solution.

32. Composition pharmaceutique suivant la revendication 30, sous forme d'un comprimé.

33. Agent antifongique utile pour le traitement d'infections fongiques chez des êtres humains, des animaux et des plantes, comprenant un composé suivant l'une quelconque des revendications 7 à 25.
